# EUROPEAN PATENT SPECIFICATION

(11) **EP 4 244 911 B1**
(45) Date of publication and mention of the grant of the patent: **03.09.2025**
(21) Application number: 21815128.0
(22) Date of filing: 12.11.2021
(51) Int. Cl.: H10K 85/30, H10K 50/17

(54) **ORGANIC ELECTRONIC DEVICE COMPRISING A COMPOUND OF FORMULA (1), DISPLAY DEVICE COMPRISING THE ORGANIC ELECTRONIC DEVICE AS WELL AS COMPOUNDS OF FORMULA (1) FOR USE IN ORGANIC ELECTRONIC DEVICES**
ORGANISCHE ELEKTRONISCHE VORRICHTUNG MIT EINER VERBINDUNG DER FORMEL (1), ANZEIGEVORRICHTUNG MIT DER ORGANISCHEN ELEKTRONISCHEN VORRICHTUNG SOWIE VERBINDUNGEN DER FORMEL (1) ZUR VERWENDUNG IN ORGANISCHEN ELEKTRONISCHEN VORRICHTUNGEN
DISPOSITIF ÉLECTRONIQUE ORGANIQUE COMPRENANT UN COMPOSÉ DE FORMULE (1), DISPOSITIF D'AFFICHAGE COMPRENANT LE DISPOSITIF ÉLECTRONIQUE ORGANIQUE, AINSI QUE COMPOSÉS DE FORMULE (1) À UTILISER DANS DES DISPOSITIFS ÉLECTRONIQUES ORGANIQUES

(30) Priority: 16.11.2020 EP 20207867
(43) Date of publication of application: 20.09.2023
(73) Proprietor: Novaled GmbH, 01099 Dresden (DE)
(72) Inventor: UVAROV, Vladimir, 01099 Dresden (DE); HUMMERT, Markus, 01099 Dresden (DE); ROSENOW, Thomas, 01099 Dresden (DE); RUNGE, Steffen, 01099 Dresden (DE); LUSCHTINETZ, Regina, 01099 Dresden (DE); LANGGUTH, Oliver, 01099 Dresden (DE); ANGERMANN, Jens, 01099 Dresden (DE)
(74) Representative: Michalski Hüttermann & Partner Patentanwälte mbB
(86) International application number: PCT/EP2021/081575
(87) International publication number: WO 2022/101439

(56) References cited:
- EP-A1- 3 133 664
- WO-A1-97/11504
- DE-A1- 102015 121 844

## Description

### Technical Field

The present invention relates to an organic electronic device comprising a compound of formula (1) and a display device comprising the organic electronic device. The invention further relates to novel compounds of formula (1) which can be of use in organic electronic devices.

### Background Art

Organic electronic devices, such as organic light-emitting diodes OLEDs, which are selfemitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the semiconductor layer, and among them, may be affected by characteristics of metal complexes which are also contained in the semiconductor layer.

In US2018331308 (A1) an organic electronic component is disclosed. In an embodiment an organic electronic component includes at least one organic layer having a fluorinated sulfonimide metal salt of the following formula: 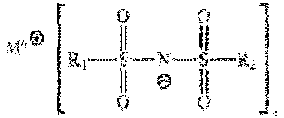 wherein M is either a divalent or higher-valent metal having an atomic mass of greater than 26 g/mol or a monovalent metal having an atomic mass of greater than or equal to 39 g/mol, where 1≤n≤7, and wherein R1, R2 are selected independently of one another from the group consisting of a fluorine-substituted aryl radical, a fluorine-substituted alkyl radical and a fluorine-substituted arylalkyl radical.

US2018342692A1 relates to an organic electronic component comprising a cathode, an anode, at least one light-emitting layer which is arranged between the anode and the cathode, a first layer, which comprises a first matrix material and a dopant, a second layer, which comprises a second matrix material, wherein the first layer is arranged between the second layer and the anode, wherein the second layer is arranged between the anode and the at least one light-emitting layer, wherein the dopant is a fluorinated sulfonimide metal salt of the following formula 1:

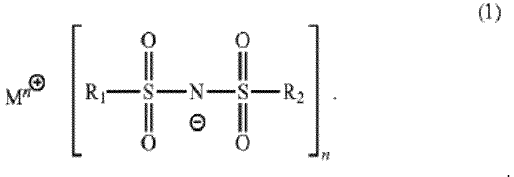

WO2017029370A1 relates to metal amides of general Formula Ia and for their use as hole injection layer (HIL) for an Organic light-emitting diode (OLED), and a method of manufacturing Organic light-emitting diode (OLED) comprising an hole injection layer containing a metal amide of general Formula Ia.

WO2017029366A1 relates to a hole injection layer for an OLED comprising a triarylamine compound doped with a charge neutral metal amide compound, characterized in that the hole injection layer has a thickness of at least about ≥ 20 nm to about ≤ 1000 nm and the charge neutral metal amide compound has the Formula Ia.

WO2018150050A1 relates to a display device comprising - a plurality of OLED pixels comprising at least two OLED pixels, the OLED pixels comprising an anode, a cathode, and a stack of organic layers, wherein the stack of organic layers - is arranged between and in contact with the cathode and the anode, and - comprises a first electron transport layer, a first hole transport layer, and a first light emitting layer provided between the first hole transport layer and the first electron transport layer, and - a driving circuit configured to separately driving the pixels of the plurality of OLED pixels, wherein, for the plurality of OLED pixels, the first hole transport layer is provided in the stack of organic layers as a common hole transport layer shared by the plurality of OLED pixels, and the first hole transport layer comprises (i) at least one first hole transport matrix compound consisting of covalently bound atoms and (ii) at least one electrical p-dopant selected from metal salts and from electrically neutral metal complexes comprising a metal cation and at least one anion and/or at least one anionic ligand consisting of at least 4 covalently bound atoms, wherein the metal cation of the electrical p-dopant is selected from alkali metals; alkaline earth metals, Pb, Mn, Fe, Co, Ni, Zn, Cd; rare earth metals in oxidation state (II) or (III); Al, Ga, In; and from Sn, Ti, Zr, Hf, V, Nb, Ta, Cr, Mo and W in oxidation state (IV) or less, a method for preparing the display device and a chemical compound for use therein.

WO 97/11504 A1 refers to a high performance battery containing an aluminum current collector which includes a noncorrosive salt disposed in a matrix, said salt being a bis(perfluoroalkylsulfonyl)imide having a total of at least 3 carbon atoms or a cyclic(perfluoroalkylenedisulfonyl)-imide.

There remains a need to improve performance of organic semiconductor materials, semiconductor layers, as well as organic electronic devices thereof, in particular to achieve improved operating voltage, improved lifetime and/or improved operating voltage stability over time through improving the characteristics of the compounds comprised therein.
Additionally, there is a need to provide compounds with improved thermal properties.

### DISCLOSURE

An aspect of the present invention provides an organic electronic device comprising an anode, a cathode, at least one photoactive layer and at least one semiconductor layer, wherein the at least one semiconductor layer is arranged between the anode and the at least one photoactive layer; and wherein the at least one semiconductor layer comprises a compound of Formula (1)

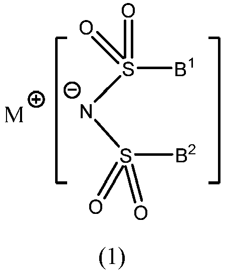

Wherein
M is Na, K, Rb or Cs,
B¹ is selected from substituted or unsubstituted isopropyl, substituted or unsubstituted C₄ to C₁₂ alkyl, substituted or unsubstituted C₆ to C₁₂ aryl, substituted or unsubstituted C₂ to C₁₂ heteroaryl,
B² is selected from substituted or unsubstituted C₁ to C₁₂ alkyl, substituted or unsubstituted C₆ to C₁₂ aryl, substituted or unsubstituted C₃ to C₁₂ heteroaryl,
wherein the substituents on B¹ and B² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, halogen, F or CN;
wherein R¹ is selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy;
wherein at least one of the substituents on B¹ and/or B² is selected from C₃ to C₉ heteroaryl, C₁ to C₆ alkoxy, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkoxy, COR¹, COOR¹, halogen, F or CN.

The negative charge in the compounds of formula (1) may be delocalised partially or fully over the N(SO₂)₂ group and optionally also over the B¹ and B² groups.

It should be noted that throughout the application and the claims any Bⁿ,Rⁿ etc. always refer to the same moieties, unless otherwise noted.

In the present specification, when a definition is not otherwise provided, "partially fluorinated" refers to an alkyl group or an alkoxy group in which only part of the hydrogen atoms are replaced by fluorine atoms.

In the present specification, when a definition is not otherwise provided, "perfluorinated" refers to an alkyl group or an alkoxy group in which all hydrogen atoms are replaced by fluorine atoms.

In the present specification, when a definition is not otherwise provided, "substituted" refers to one substituted with a deuterium, C₁ to C₁₂ alkyl and C₁ to C₁₂ alkoxy.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, an "alkyl group" refers to a saturated aliphatic hydrocarbyl group. The alkyl group may be a C₁ to C₁₂ alkyl group. More specifically, the alkyl group may be a C₁ to C₁₀ alkyl group or a C₁ to C₆ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an iso-propyl group, a butyl group, an iso-butyl group, a tert-butyl group, a pentyl group, a hexyl group.

In the context of the present invention, "ⁱCₙH₍₂ₙ₊₁₎" denotes an iso-alkyl group and "ⁱCₙF₍₂ₙ₊₁₎" denotes a perfluorinated iso-alkyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methylcyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from B, Si, N, P, O, S; more preferably from N, P, O, S.

In the present specification, "aryl group" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluorenyl.

Analogously, under heteroaryl, it is especially where suitable understood a group derived by formal abstraction of one ring hydrogen from a heterocyclic aromatic ring in a compound comprising at least one such ring.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common sp²-hybridized carbon atoms

In the present specification, the single bond refers to a direct bond.

In the context of the present invention, "different" means that the compounds do not have an identical chemical structure.

The term "free of", "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

The terms anode, anode layer and anode electrode are used synonymously.

The terms cathode, cathode layer and cathode electrode are used synonymously.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) level.

### Advantageous Effects

Surprisingly, it was found that the organic electronic device according to the invention solves the problem underlying the present invention by enabling devices in various aspects superior over the organic electroluminescent devices known in the art, in particular with respect to operating voltage over lifetime, improved lifetime and/or improved voltage stability over time.

According to one embodiment of the present invention, the compound of formula (1) comprises at least four carbon atoms and at least five fluorine atoms.

According to one embodiment of the present invention, the compound of formula (1) comprises at least six carbon atoms and at least eight fluorine atoms.

According to a preferred embodiment of the present invention, M is Na, K or Cs.

According to a preferred embodiment of the present invention, M is Na.

According to one embodiment of the present invention, the substituents on B¹ and B² are independently selected from halogen, with F especially preferred, C₁ to C₃ perhalogenated, especially perfluorinated alkyl or alkoxy, or -(O)ₗ-CₘH₂ₘ-CₙHalₙ₂ₙ₊₁ with l= 0 or 1, especially 0, m = 1 or 2, especially 1 and n = 1 to 3, especially 1 or 2 and Hal= halogen, especially F.

According to one embodiment of the present invention, at least one of B¹ and B² is substituted alkyl and the substituents of the alkyl moiety are fluorine with the number n_{F} (of fluorine substituents) and n_{H} (of hydrogens) follow the equation: n_{F} > n_{H} + 2.

According to one embodiment of the present invention, M is Na, K or Cs; B¹ is selected from substituted or unsubstituted isopropyl, substituted or unsubstituted C₄ to C₆ alkyl, substituted or unsubstituted phenyl, substituted or unsubstituted C₃ to C₅ heteroaryl; B² is selected from substituted or unsubstituted C₁ to C₆ alkyl, substituted or unsubstituted phenyl, substituted or unsubstituted C₃ to C₅ heteroaryl; wherein at least one of the substituents on B¹ and/or B² is selected from partially or perfluorinated C₁ to C₄ alkyl, halogen, F or CN; wherein the sum of substituents is at least two.

According to one embodiment of the present invention, M is Na; B¹ is selected from substituted or unsubstituted isopropyl, substituted or unsubstituted C₄ to C₆ alkyl, substituted or unsubstituted phenyl, substituted or unsubstituted C₃ to C₅ heteroaryl; B² is selected from substituted or unsubstituted C₁ to C₆ alkyl, substituted or unsubstituted phenyl, substituted or unsubstituted C₃ to C₅ heteroaryl; wherein at least one of the substituents on B¹ and/or B² is selected from partially or perfluorinated C₁ to C₄ alkyl, halogen, F or CN; wherein the sum of substituents is at least two.

According to one embodiment of the present invention, M is selected Na, K or Cs; B¹ is selected from substituted or unsubstituted isopropyl, substituted or unsubstituted C₄ alkyl, substituted or unsubstituted phenyl, substituted or unsubstituted C₃ to C₅ heteroaryl; B² is selected from substituted or unsubstituted C₁ to C₆ alkyl, substituted or unsubstituted phenyl, substituted or unsubstituted C₃ to C₅ heteroaryl; wherein at least one of the substituents on B¹ and/or B² is selected from CF₃, F or CN; wherein the sum of substituents is at least five.

According to one embodiment of the present invention, M is selected Na; B¹ is selected from substituted or unsubstituted isopropyl, substituted or unsubstituted C₄ alkyl, substituted or unsubstituted phenyl, substituted or unsubstituted C₃ to C₅ heteroaryl; B² is selected from substituted or unsubstituted C₁ to C₆ alkyl, substituted or unsubstituted phenyl, substituted or unsubstituted C₃ to C₅ heteroaryl; wherein at least one of the substituents on B¹ and/or B² is selected from CF₃, F or CN; wherein the sum of substituents is at least five.

According to one embodiment of the present invention, at least one of B¹ and B² is selected from perfluorinated alkyl or pentafluorophenyl.

According to one embodiment of the present invention, the compound of formula (1) does not contain hydrogen.

According to one embodiment of the present invention, both of B¹ and B² are selected from perfluorinated alkyl or pentafluorophenyl.

According to one embodiment of the present invention, the compound of formula (1) contains 12 carbon atoms or less.

According to one embodiment of the present invention, the compound of formula (1) contains 20 fluorine atoms or less.

According to one embodiment of the present invention, compound of formula (1) is free of alkoxy, COR¹ and/or COOR¹ groups.

According to one embodiment of the present invention, B¹ and B² are identical.

According to one embodiment of the present invention B¹ and B² are not identical.

According to one embodiment of the present invention, one of B¹ and B² is pentafluorophenyl, whereas the other is a different group.

According to one embodiment of the present invention, one of B¹ and B² is methyl, whereas the other is a different group.

According to one embodiment, the anion in compound of formula (1) is selected from the anions A-1 to A-51: 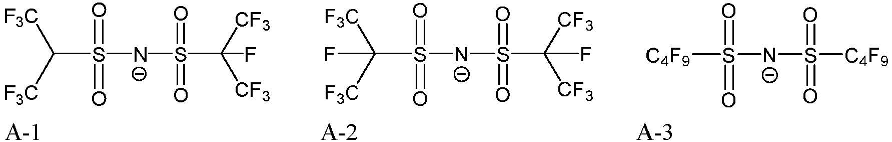 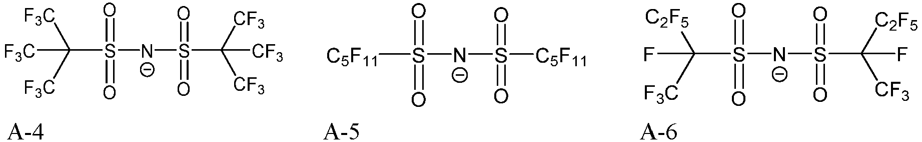 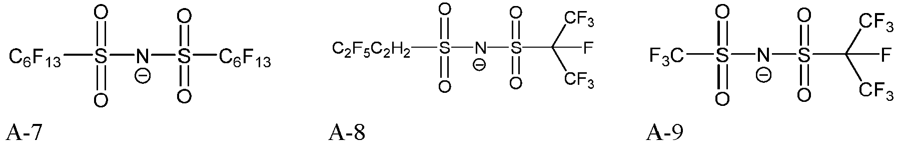 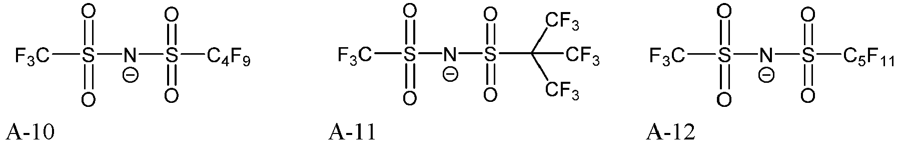 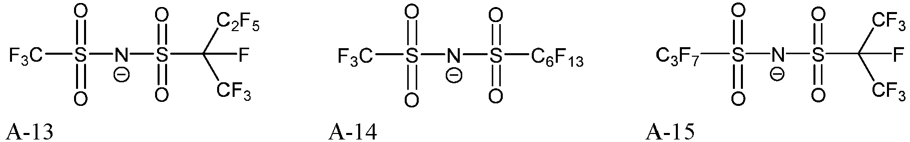 A-16 A-17 A-18 A-19 A-20 A-21 A-22 A-23 A-24 A-25 A-26 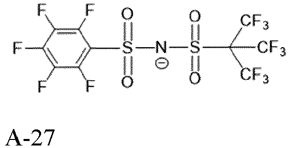 A-28 A-29 A-30 A-31 A-32 A-33 A-34 A-35 A-36 A-37 A-38 A-39

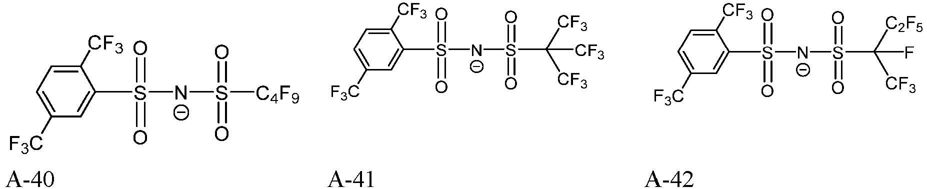

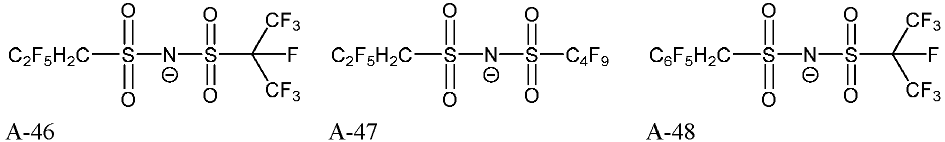

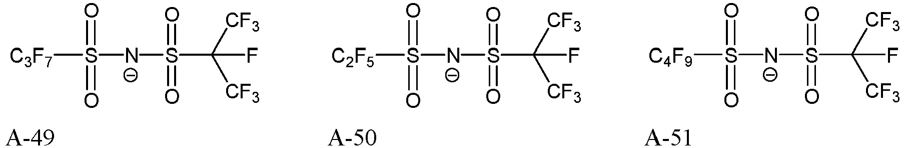

According to one embodiment of the present invention, M is Na and the anion in compound of formula (1) is selected from the anions A-1 to A-51.

According to one embodiment of the present invention the compound of formula (1) is selected from the compounds A1 to A12

| Name | Structure |
|---|---|
| A1 | 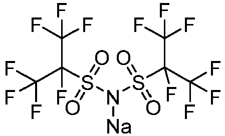 |
| A2 | 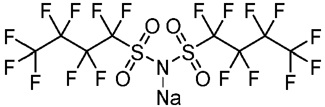 |
| A3 | 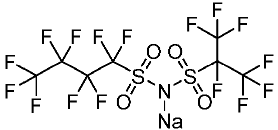 |
| A4 | 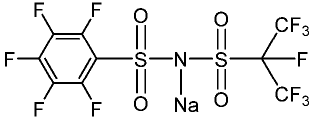 |
| A5 | 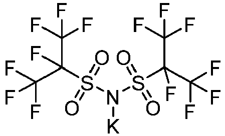 |
| A6 | 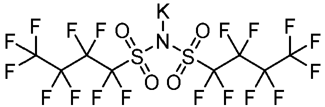 |
| A7 | 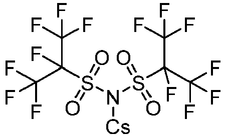 |
| A8 | 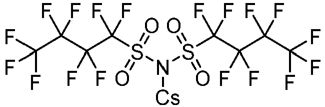 |
| A9 | 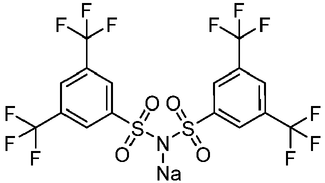 |
| A10 | 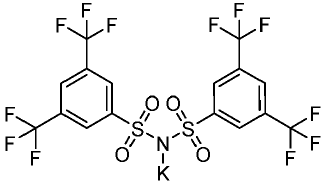 |
| A11 | 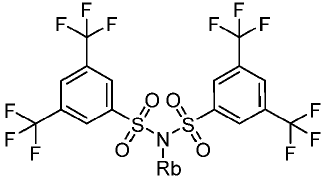 |
| A12 | 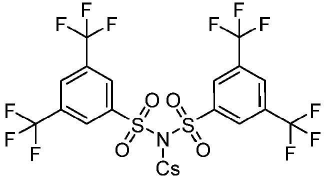 |

According to one embodiment of the present invention the semiconductor layer and/or the compound of formula (1) are non-emissive.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about ≥ 380 nm to about ≤ 780 nm.

According to one embodiment of the invention, at least one semiconductor layer is arranged and/or provided adjacent to the anode layer.

According to one embodiment of the invention, at least one semiconductor layer of the present invention is a hole-injection layer.

In case the at least one semiconductor layer of the present invention is a hole-injection layer and/ or is arranged and/or provided adjacent to the anode layer then it is especially preferred that this layer consists essentially of the compound of formula (1).

In the context of the present specification the term "consisting essentially of " especially means and/or includes a concentration of ≥ 90% (vol/vol) more preferred ≥ 95% (vol/vol) and most preferred ≥ 99% (vol/vol).

According to another aspect, the at least one semiconductor layer may have a layer thickness of at least about ≥ 0.5 nm to about ≤ 10 nm, preferably of about ≥ 2 nm to about ≤ 8 nm, also preferred of about ≥ 3 nm to about ≤ 5 nm.

According to one embodiment of the invention, at least one semiconductor layer of the present invention further comprises a substantially covalent matrix compound. Preferably at least one semiconductor layer further comprising a substantially covalent matrix compound is arranged and/or provided adjacent to the anode layer.

### Substantially covalent matrix compound

The organic semiconductor layer may further comprises a substantially covalent matrix compound. According to one embodiment the substantially covalent matrix compound may be selected from at least one organic compound. The substantially covalent matrix may consists substantially from covalently bound C, H, O, N, S, which optionally comprise in addition covalently bound B, P, As and/or Se.

According to one embodiment of the organic electronic device, the organic semiconductor layer further comprises a substantially covalent matrix compound, wherein the substantially covalent matrix compound may be selected from organic compounds consisting substantially from covalently bound C, H, O, N, S, which optionally comprise in addition covalently bound B, P, As and/or Se.

Organometallic compounds comprising covalent bonds carbon-metal, metal complexes comprising organic ligands and metal salts of organic acids are further examples of organic compounds that may serve as substantially covalent matrix compounds of the hole injection layer.

In one embodiment, the substantially covalent matrix compound lacks metal atoms and majority of its skeletal atoms may be selected from C, O, S, N. Alternatively, the substantially covalent matrix compound lacks metal atoms and majority of its skeletal atoms may be selected from C and N.

According to one embodiment, the substantially covalent matrix compound may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

Preferably, the substantially covalent matrix compound comprises at least one arylamine moiety, alternatively a diarylamine moiety, alternatively a triarylamine moiety.

In one embodiment, the HOMO level of the substantially covalent matrix compound may be more negative than the HOMO level of N2,N2,N2',N2',N7,N7,N7',N7'-octakis(4-methoxyphenyl)-9,9'-spirobi[fluorene]-2,2',7,7'-tetraamine (CAS 207739-72-8) when determined under the same conditions.

In one embodiment, the HOMO level of the substantially covalent matrix compound may be more negative than the HOMO level of N,N'-Bis(naphthalen-1-yl)-N,N'-bis(phenyl)-benzidine (CAS 123847-85-8) when determined under the same conditions.

In one embodiment, the HOMO level of the substantially covalent matrix compound may be more negative than the HOMO level of N-([1,1'-biphenyl]-4-yl)-9,9-dimethyl-N-(4-(9-phenyl-9H-carbazol-3-yl)phenyl)-9H-fluoren-2-amine (CAS 1242056-42-3) when determined under the same conditions.

In one embodiment, the HOMO level of the substantially covalent matrix compound , when calculated using TURBOMOLE V6.5 (TURBOMOLE GmbH, Litzenhardtstrasse 19, 76135 Karlsruhe, Germany) by applying the hybrid functional B3LYP with a 6-31G* basis set in the gas phase, may be more negative than -4.27 eV, preferably more negative than -4.3 eV, alternatively more negative than -4.5 eV, alternatively more negative than -4.6 eV, alternatively more negative than -4.65 eV.

In one embodiment, the HOMO level of the substantially covalent matrix compound may be more negative than the HOMO level of N2,N2,N2',N2',N7,N7,N7',N7'-octakis(4-methoxyphenyl)-9,9'-spirobi[fluorene]-2,2',7,7'-tetraamine (CAS 207739-72-8) and more positive than the HOMO level of N-([1,1'-biphenyl]-4-yl)-N-(2-(9,9-diphenyl-9H-fluoren-4-yl)phenyl)-9,9-dimethyl-9H-fluoren-2-amine when determined under the same conditions.

In one embodiment of the present invention, the substantially covalent matrix compound may be free of alkoxy groups.

In one embodiment, the HOMO level of the substantially covalent matrix compound, when calculated using TURBOMOLE V6.5 (TURBOMOLE GmbH, Litzenhardtstrasse 19, 76135 Karlsruhe, Germany) by applying the hybrid functional B3LYP with a 6-31G* basis set in the gas phase, may be selected in the range of < -4.27 eV and ≥ -4.84 eV, alternatively in the range of < - 4.3 eV and ≥ -4.84 eV, alternatively in the range of < -4.5 eV and ≥ -4.84 eV, alternatively in the range of < -4.5 eV and ≥ -4.84 eV, alternatively in the range of < -4.6 eV and ≥ -4.84 eV.

In the context of the present specification, "more negative" means that the absolute value is more positive than the comparative value.

Preferably, the substantially covalent matrix compound is free of metals and/or ionic bonds.

### Compound of formula (VI) or a compound of formula (VII)

According to another aspect of the present invention, the at least one matrix compound, also referred to as "substantially covalent matrix compound", may comprises at least one arylamine compound, diarylamine compound, triarylamine compound, a compound of formula (VI) or a compound of formula (VII): 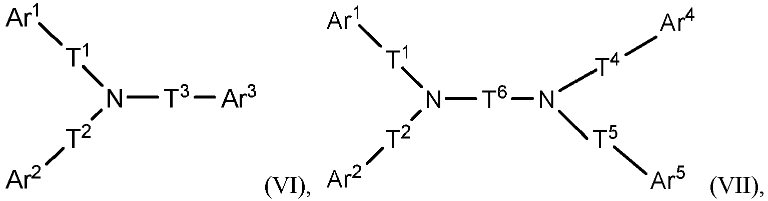 wherein:
T¹, T², T³, T⁴ and T⁵ are independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ are independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein
the substituents of Ar¹, Ar², Ar³, Ar⁴ and Ar⁵ are selected the same or different from the group comprising H, D, F, C(-O)R², CN, Si(R²)₃, P(-O)(R²)₂, OR², S(-O)R², S(-O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted C₆ to C₁₈ aryl, unsubstituted C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
wherein R² may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl or C₃ to C₁₈ heteroaryl.

According to an embodiment wherein T¹, T², T³, T⁴ and T⁵ may be independently selected from a single bond, phenylene, biphenylene or terphenylene. According to an embodiment wherein T¹, T², T³, T⁴ and T⁵ may be independently selected from phenylene, biphenylene or terphenylene and one of T¹, T², T³, T⁴ and T⁵ are a single bond. According to an embodiment wherein T¹, T², T³, T⁴ and T⁵ may be independently selected from phenylene or biphenylene and one of T¹, T², T³, T⁴ and T⁵ are a single bond. According to an embodiment wherein T¹, T², T³, T⁴ and T⁵ may be independently selected from phenylene or biphenylene and two of T¹, T², T³, T⁴ and T⁵ are a single bond.

According to an embodiment wherein T¹, T² and T³ may be independently selected from phenylene and one of T¹, T² and T³ are a single bond. According to an embodiment wherein T¹, T² and T³ may be independently selected from phenylene and two of T¹, T² and T³ are a single bond.

According to an embodiment wherein T⁶ may be phenylene, biphenylene, terphenylene. According to an embodiment wherein T⁶ may be phenylene. According to an embodiment wherein T⁶ may be biphenylene. According to an embodiment wherein T⁶ may be terphenylene.

According to an embodiment wherein Ar¹, Ar², Ar³, Ar⁴ and Ar⁵ may be independently selected from D1 to D16: 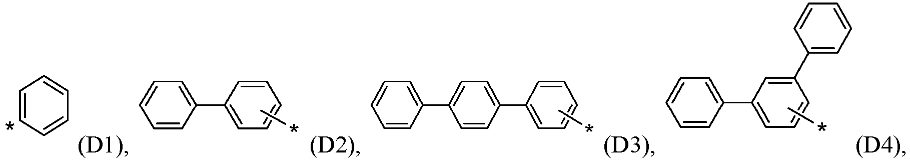 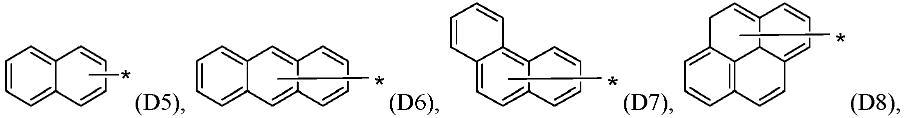 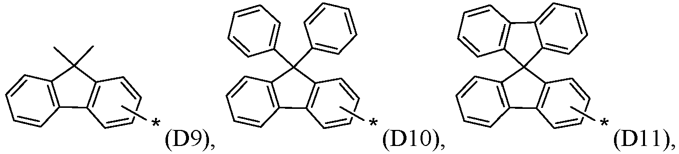 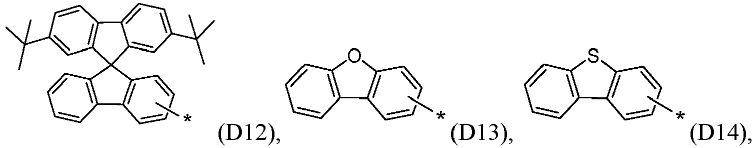 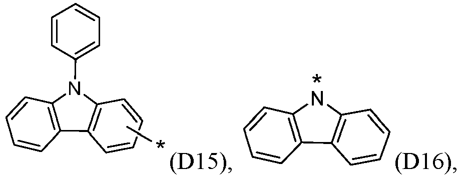 wherein the asterix "*" denotes the binding position.

According to an embodiment, wherein Ar¹, Ar², Ar³, Ar⁴ and Ar⁵ may be independently selected from D1 to D15; alternatively selected from D1 to D10 and D13 to D15.

According to an embodiment, wherein Ar¹, Ar², Ar³, Ar⁴ and Ar⁵ may be independently selected from the group consisting of D1, D2, D5, D7, D9, D10, D13 to D16.

The rate onset temperature may be in a range particularly suited to mass production, when Ar¹, Ar², Ar³, Ar⁴ and Ar⁵ are selected in this range.

The "matrix compound of formula (VI) or formula (VII)" may be also referred to as "hole transport compound".

According to one embodiment, the substantially covalent matrix compound comprises at least one naphthyl group, carbazole group, dibenzofurane group, dibenzothiophene group and/or substituted fluorenyl group, wherein the substituents are independently selected from methyl, phenyl or fluorenyl.

According to an embodiment of the electronic device, wherein the matrix compound of formula (VI) or formula (VII) are selected from F1 to F17:

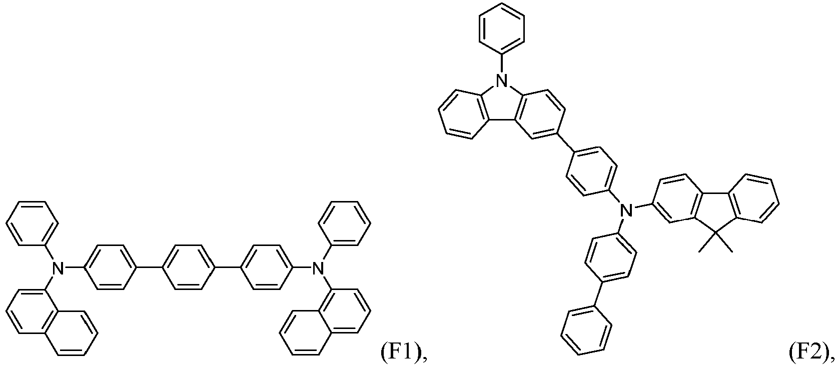

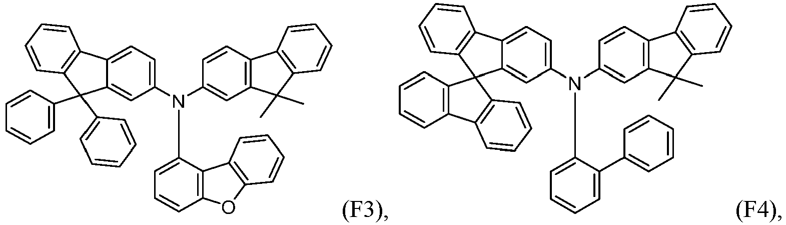

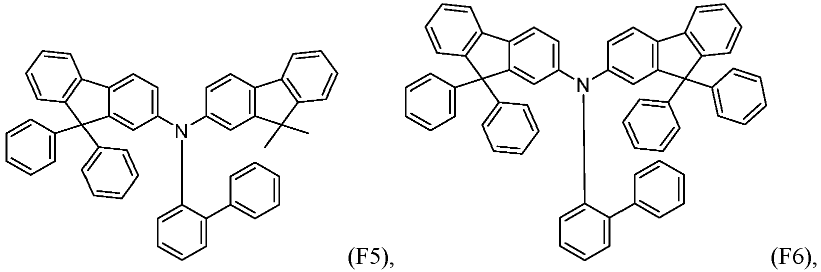

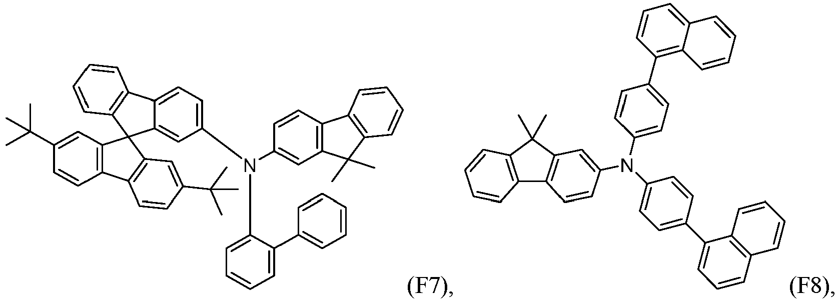

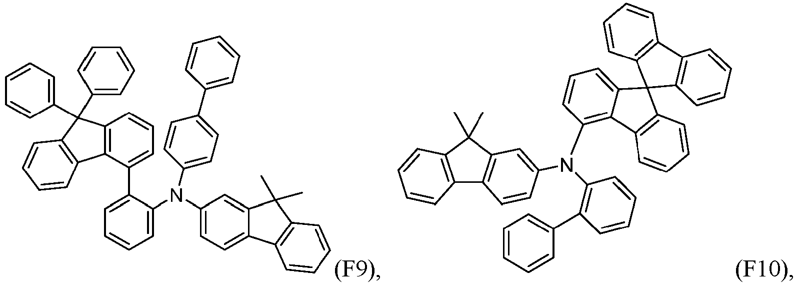

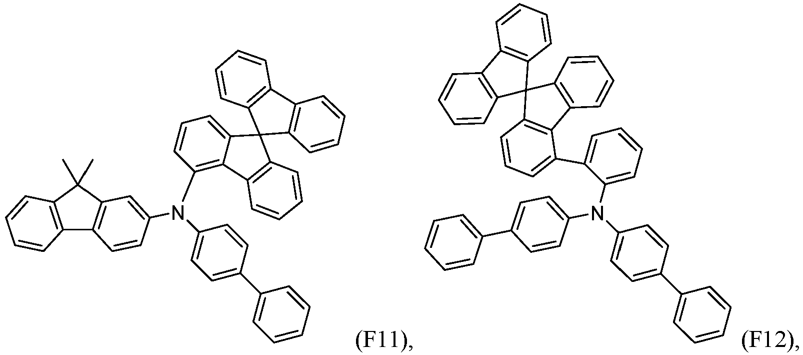

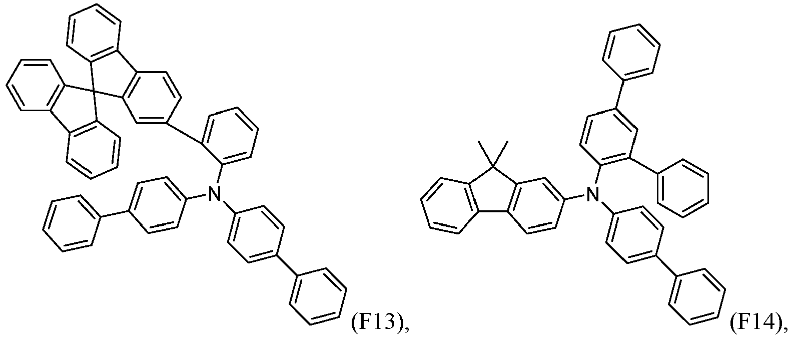

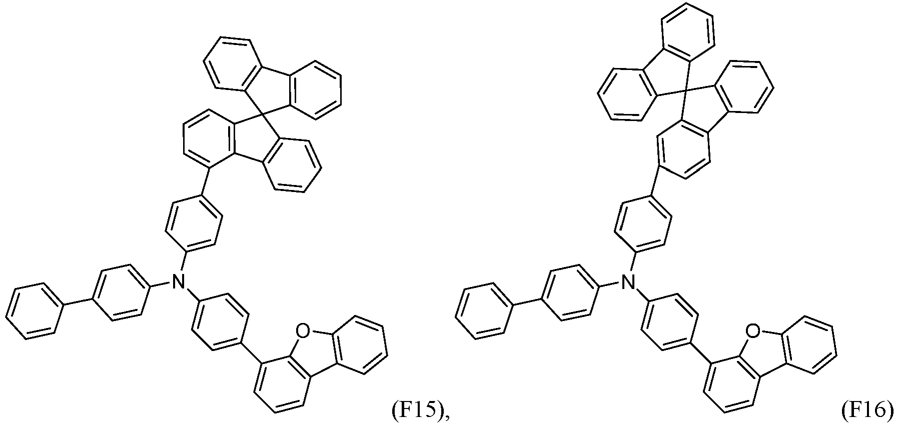

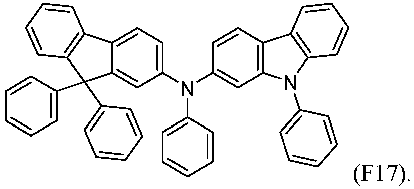

The substantially covalent matrix compound may be free of HTM014, HTM081, HTM163, HTM222, EL-301, HTM226, HTM355, HTM133, HTM334, HTM604 and/or EL-22T. The abbreviations denote the manufacturers' names, for example, of Merck or Lumtec.

### Organic semiconductor layer

The organic semiconductor layer may be formed on the anode layer or cathode layer by vacuum deposition, spin coating, printing, casting, slot-die coating, Langmuir-Blodgett (LB) deposition, or the like. When the Organic semiconductor layer is formed using vacuum deposition, the deposition conditions may vary according to the compound(s) that are used to form the layer, and the desired structure and thermal properties of the layer. In general, however, conditions for vacuum deposition may include a deposition temperature of 100° C to 350° C, a pressure of 10⁻⁸ to 10⁻³ Torr (1 Torr equals 133.322 Pa), and a deposition rate of 0.1 to 10 nm/sec.

When the organic semiconductor layer is formed using spin coating or printing, coating conditions may vary according to the compound(s) that are used to form the layer, and the desired structure and thermal properties of the organic semiconductor layer. For example, the coating conditions may include a coating speed of about 2000 rpm to about 5000 rpm, and a thermal treatment temperature of about 80° C to about 200° C. Thermal treatment removes a solvent after the coating is performed.

The thickness of the organic semiconductor layer may be in the range from about 1 nm to about 20 nm, and for example, from about 2 nm to about 15 nm, alternatively about 2 nm to about 12 nm.

When the thickness of the organic semiconductor layer is within this range, the organic semiconductor layer may have excellent hole injecting and/or hole generation characteristics, without a substantial penalty in driving voltage.

According to one embodiment of the present invention, the organic semiconductor layer may comprise:
- at least about ≥ 0.5 wt.-% to about ≤ 30 wt.-%, preferably about ≥ 0.5 wt.-% to about ≤ 20 wt.-%, and more preferred about ≥ 1 wt.-% to about ≤ 15 wt.-% of a compound of formula (1), and
- at least about ≥ 70 wt.-% to about ≤ 99.5 wt.-%, preferably about ≥ 80 wt.-% to about ≤ 99.5 wt.-%, and more preferred about ≥ 85 wt.-% to about ≤ 99 wt.-% of a substantially covalent matrix compound; preferably the wt.-% of the compound of formula (1) is lower than the wt.-% of the substantially covalent matrix compound; wherein the weight-% of the components are based on the total weight of the organic semiconductor layer.

According to one embodiment of the invention, the organic electronic device comprises at least one photoactive layer and the at least one of the at least one organic semiconductor layers is arranged between the anode and the at least one photoactive layer.

According to one embodiment of the invention, the organic electronic device comprises at least two photoactive layers, wherein at least one of the at least one organic semiconductor layers is arranged between the first and the second photoactive layer.

According to one embodiment of the invention, the organic electronic devices comprises at least one photoactive layer, wherein the photoactive layer is arranged between the anode layer and the cathode layer.

According to one embodiment of the invention, the organic electronic devices comprises at least one photoactive layer and the at least one organic semiconductor layers is arranged between the anode and the at least one photoactive layer.

According to one embodiment of the invention, the organic electronic device comprises at least two photoactive layers, wherein at least one of the at least one organic semiconductor layers is arranged between the first and the second photoactive layer.

According to one embodiment of the invention, the organic electronic device comprises at least two photoactive layers, wherein one of the at least one organic semiconductor layers is arranged between the first and the second photoactive layer and one of the at least one organic semiconductor layers is arranged between the anode layer and the first photoactive layer.

According to one embodiment of the invention, the electronic organic device is an electroluminescent device, preferably an organic light emitting diode.

According to one embodiment of the invention, the electronic organic device is an electroluminescent device, preferably an organic light emitting diode and the light is emitted through the cathode layer.

The present invention furthermore relates to a display device comprising an organic electronic device according to the present invention.

According to one embodiment of the invention the electronic organic device is an electroluminescent device, preferably an organic light emitting diode.

The present invention furthermore relates to a display device comprising an organic electronic device according to the present invention.

The present invention furthermore relates to a compound of Formula (1): 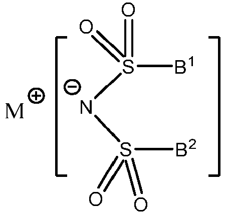 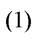 wherein Formula (1) is represented by one of the following formulae (1a) or (1b):

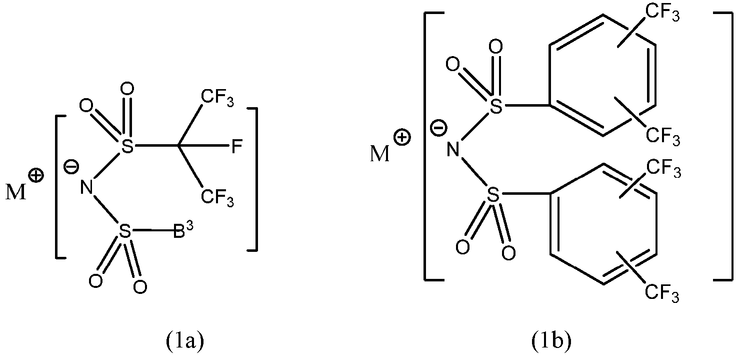

Wherein
M is Na, K, Rb or Cs,
B³ is selected from substituted or unsubstituted C₁ to C₁₂ alkyl, substituted or unsubstituted C₆ to C₁₂ aryl, substituted or unsubstituted C₃ to C₁₂ heteroaryl,
wherein the substituents on B³ are independently selected from D, C₆ aryl, C₅ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, halogen, F or CN;
wherein R¹ is selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy.

According to a preferred embodiment the compound is selected from formula (1a),

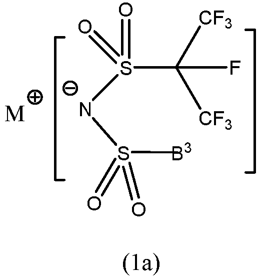

Wherein
M is Na, K, Rb or Cs,
B³ is selected from substituted or unsubstituted C₁ to C₁₂ alkyl, substituted or unsubstituted C₆ to C₁₂ aryl, substituted or unsubstituted C₃ to C₁₂ heteroaryl,
wherein the substituents on B³ are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, halogen, F or CN;
wherein R¹ is selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy.

The negative charge in compounds of formula (1a) may be delocalised partially or fully over the N(SO₂)₂ group and optionally also over the B¹ and B² groups.

According to a preferred embodiment of the present invention, M is Na, K or Cs; B³ is selected from substituted or unsubstituted C₁ to C₆ alkyl, substituted or unsubstituted phenyl, substituted or unsubstituted C₃ to C₅ heteroaryl; wherein the substituents on B³ are independently selected from partially or perfluorinated C₁ to C₄ alkyl, halogen, F or CN.

According to a preferred embodiment of the present invention, M is Na; B³ is selected from substituted or unsubstituted C₁ to C₆ alkyl, substituted or unsubstituted phenyl, substituted or unsubstituted C₃ to C₅ heteroaryl; wherein the substituents on B³ are independently selected from partially or perfluorinated C₁ to C₄ alkyl, halogen, F or CN.

Any specifications of formula (1) as described above in the context of the organic electronic device apply *mutatis mutandis* wherever possible.

Preferably the compound of formula (1a) is selected from the group comprising the following structures:

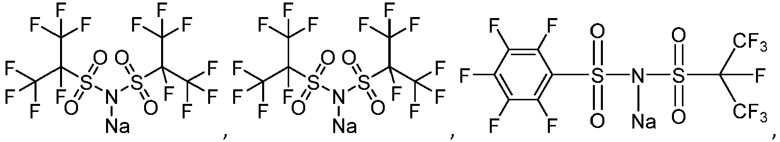

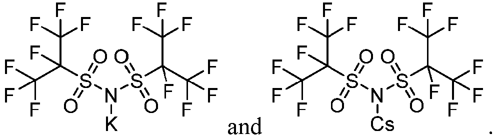

According to a preferred embodiment the compound is selected from formula (1b),

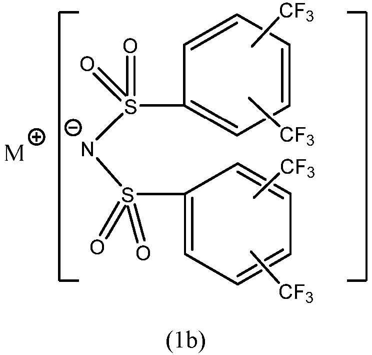

Wherein
M is Na, K, Rb or Cs.

According to a preferred embodiment, the compound of formula (1b) is selected from

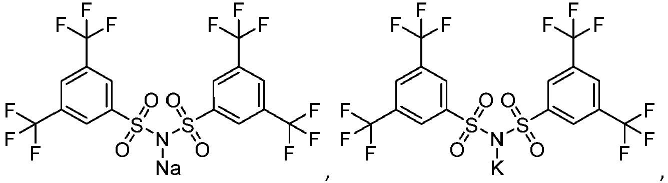

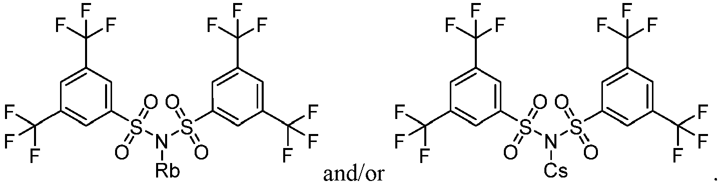

The negative charge in compounds of formula (1b) may be delocalised partially or fully over the N(SO₂)₂ group and optionally also over the phenyl groups.

Any specifications of formula (1) as described above in the context of the organic electronic device apply *mutatis mutandis* wherever possible.

### Further layers

In accordance with the invention, the organic electronic device may comprise, besides the layers already mentioned above, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

### Anode layer

The anode layer may be formed by depositing or sputtering a material that is used to form the anode electrode. The material used to form the anode electrode may be a high work-function material, so as to facilitate hole injection. The anode material may also be selected from a low work function material (i.e. aluminum). The anode layer may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode layer may also be formed using metals, typically silver (Ag), gold (Au), or metal alloys.

According to a preferred embodiment the organic electrode device comprises an anode layer, whereby the anode layer comprises a first anode sub-layer and a second anode sub-layer, wherein
- the first anode sub-layer comprises a first metal having a work function in the range of ≥ 4 and ≤ 6 eV, and
- the second anode sub-layer comprises a transparent conductive oxide; and
- the second anode sub-layer is arranged closer to the hole injection layer.

According to one embodiment of the present invention, the first metal of the first anode sub-layer may be selected from the group comprising Ag, Mg, Al, Cr, Pt, Au, Pd, Ni, Nd, Ir, preferably Ag, Au or Al, and more preferred Ag.

According to one embodiment of the present invention, the first anode sub-layer has have a thickness in the range of 5 to 200 nm, alternatively 8 to 180 nm, alternatively 8 to 150 nm, alternatively 100 to 150 nm.

According to one embodiment of the present invention, the first anode sub-layer is formed by depositing the first metal via vacuum thermal evaporation.

It is to be understood that the first anode layer is not part of the substrate.

According to one embodiment of the present invention, the transparent conductive oxide of the second anode sub layer is selected from the group selected from the group comprising indium tin oxide or indium zinc oxide, more preferred indium tin oxide.

According to one embodiment of the present invention, the second anode sub-layer may has a thickness in the range of 3 to 200 nm, alternatively 3 to 180 nm, alternatively 3 to 150 nm, alternatively 3 to 20 nm.

According to one embodiment of the present invention, the second anode sub-layer may be formed by sputtering of the transparent conductive oxide.

According to one embodiment of the present invention, anode layer of the organic electronic device comprises in addition a third anode sub-layer comprising a transparent conductive oxide, wherein the third anode sub-layer is arranged between the substrate and the first anode sub-layer.

According to one embodiment of the present invention, the third anode sub-layer comprises a transparent oxide, preferably from the group selected from the group comprising indium tin oxide or indium zinc oxide, more preferred indium tin oxide.

According to one embodiment of the present invention, the third anode sub-layer may has a thickness in the range of 3 to 200 nm, alternatively 3 to 180 nm, alternatively 3 to 150 nm, alternatively 3 to 20 nm.

According to one embodiment of the present invention, the third anode sub-layer may be formed by sputtering of the transparent conductive oxide.

It is to be understood that the third anode layer is not part of the substrate.

According to one embodiment of the present invention, the hole injection layer is in direct contact with the anode layer.

### Hole injection layer

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10⁻⁸ to 10⁻³ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a holetransporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; α-NPD (N,N'-Bis(naphthalen-1-yl)-N,N'-bis(phenyl)-benzidine) doped with F4TCNQ. α-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-1-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

According to one embodiment of the present invention, the hole transport layer may comprise the same substantially covalent matrix compound as the semiconductor layer of the present invention.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime may be improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

### Photoactive layer (PAL)

The photoactive layer converts an electrical current into photons or photons into an electrical current.

The PAL may be formed on the HTL by vacuum deposition, spin coating, slot-die coating, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL.

It may be provided that the photoactive layer does not comprise the compound of Formula (1).

The photoactive layer may be a light-emitting layer or a light-absorbing layer.

### Emission layer (EML)

The EML may be formed on the HTL by vacuum deposition, spin coating, slot-die coating, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

It may be provided that the emission layer does not comprise the compound of Formula (1).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4''-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and triazine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

### Electron transport layer (ETL)

The organic electronic device according to the present invention may further comprise an electron transport layer (ETL).

According to another embodiment of the present invention, the electron transport layer may further comprise an azine compound, preferably a triazine compound.

In one embodiment, the electron transport layer may further comprise a dopant selected from an alkali organic complex, preferably LiQ.

The thickness of the ETL may be in the range from about 15 nm to about 50 nm, for example, in the range from about 20 nm to about 40 nm. When the thickness of the EIL is within this range, the ETL may have satisfactory electron-injecting properties, without a substantial penalty in driving voltage.

According to another embodiment of the present invention, the organic electronic device may further comprise a hole blocking layer and an electron transport layer, wherein the hole blocking layer and the electron transport layer comprise an azine compound. Preferably, the azine compound is a triazine compound.

### Electron injection layer (EIL)

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL.

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

### Cathode electrode

The cathode electrode is formed on the ETL or optional EIL. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed from a metal or metal alloy.

It is to be understood that the cathode electrode is not part of an electron injection layer or the electron transport layer.

### Organic light-emitting diode (OLED)

The organic electronic device according to the invention may be an organic light-emitting device.

According to one aspect of the present invention, there is provided an organic light-emitting diode (OLED) comprising: a substrate; an anode electrode formed on the substrate; an semiconductor layer comprising compound of formula (1) , a hole transport layer, an emission layer, an electron transport layer and a cathode electrode.

According to another aspect of the present invention, there is provided an OLED comprising: a substrate; an anode electrodelayer formed on the substrate; a semiconductor layer comprising a compound of Formula (1), a hole transport layer, an electron blocking layer, an emission layer, a hole blocking layer, an electron transport layer and a cathode layer.

According to another aspect of the present invention, there is provided an OLED comprising: a substrate; an anode layer formed on the substrate; a semiconductor layer comprising a compound of Formula (1), a hole transport layer, an electron blocking layer, an emission layer, a hole blocking layer, an electron transport layer, an electron injection layer, and a cathode layer.

According to various embodiments of the present invention, there may be provided OLEDs layers arranged between the above mentioned layers, on the substrate or on the top layer.

According to one aspect, the OLED may comprise a layer structure of a substrate that is adjacent arranged to an anode layer, the anode layer is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a hole generating layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode layer an optional electron transport layer and/or an optional injection layer are arranged.

The semiconductor layer according to the invention may be the first hole injection layer and p-type charge generation layer.

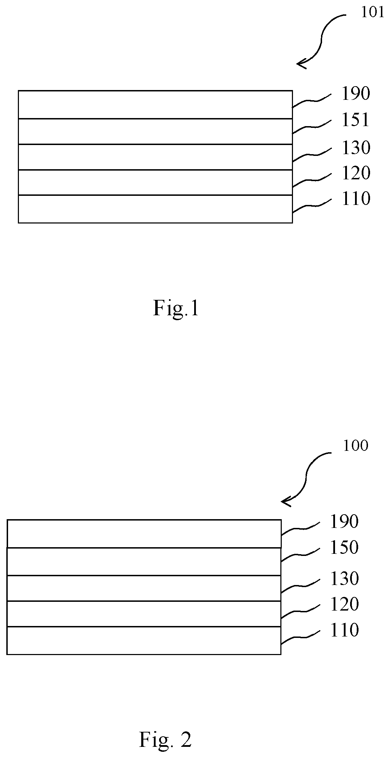

For example, the OLED according to Fig. 2 may be formed by a process, wherein on a substrate (110), an anode layer (120), a hole injection layer (130), a hole transport layer (140), an electron blocking layer (145), an emission layer (150), a hole blocking layer (155), an electron transport layer (160), an electron injection layer (180) and the cathode layer (190) are subsequently formed in that order.

### Organic electronic device

The organic electronic device according to the invention may be a light emitting device, or a photovoltaic cell, and preferably a light emitting device.

According to another aspect of the present invention, there is provided a method of manufacturing an organic electronic device, the method using:
- at least one deposition source, preferably two deposition sources and more preferred at least three deposition sources.

The methods for deposition that can be suitable comprise:
- deposition via vacuum thermal evaporation;
- deposition via solution processing, preferably the processing is selected from spincoating, printing, casting; and/or
- slot-die coating.

According to various embodiments of the present invention, there is provided a method using:
- a first deposition source to release the compound of Formula (1) according to the invention, and
- a second deposition source to release the substantially covalent matrix compound;
the method comprising the steps of forming the semiconductor layer; whereby for an organic light-emitting diode (OLED):
- the semiconductor layer is formed by releasing the compound of Formula (1) according to the invention from the first deposition source and the substantially covalent matrix compound from the second deposition source.

According to various embodiments of the present invention, the method may further include forming on the anode layer, at least one layer selected from the group consisting of forming a hole transport layer or forming a hole blocking layer, and an emission layer between the anode layer and the first electron transport layer.

According to various embodiments of the present invention, the method may further include the steps for forming an organic light-emitting diode (OLED), wherein
- on a substrate an anode layer is formed,
- on the anode layer a semiconductor layer comprising a compound of formula (1) is formed,
- on the semiconductor layer comprising a compound of formula (1) a hole transport layer is formed,
- on the hole transport layer an emission layer is formed,
- on the emission layer an electron transport layer is formed, optionally a hole blocking layer is formed on the emission layer,
- and finally a cathode layer is formed,
- optional a hole blocking layer is formed in that order between the first anode layer and the emission layer,
- optional an electron injection layer is formed between the electron transport layer and the cathode layer.

According to various embodiments, the OLED may have the following layer structure, wherein the layers having the following order:
anode, semiconductor layer comprising a compound of Formula (1) according to the invention, first hole transport layer, second hole transport layer, emission layer, optional hole blocking layer, electron transport layer, optional electron injection layer, and cathode.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

Hereinafter, the embodiments are illustrated in more detail with reference to examples. However, the present disclosure is not limited to the following examples. Reference will now be made in detail to the exemplary aspects.

### Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

### Figures 1 to 6

FIG. 1 is a schematic sectional view of an organic electronic device, according to an exemplary embodiment of the present invention;
FIG. 2 is a schematic sectional view of an organic light-emitting diode (OLED), according to an exemplary embodiment of the present invention;
FIG. 3 is a schematic sectional view of an organic light-emitting diode (OLED), according to an exemplary embodiment of the present invention.
FIG. 4 is a schematic sectional view of an organic light-emitting diode (OLED), according to an exemplary embodiment of the present invention;
FIG. 5 is a schematic sectional view of an organic light-emitting diode (OLED), according to an exemplary embodiment of the present invention.
FIG. 6 is a schematic sectional view of an organic light-emitting diode (OLED), according to an exemplary embodiment of the present invention.

Hereinafter, the figures 1 to 6 are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic electronic device 101, according to an exemplary embodiment of the present invention. The organic electronic device 101 includes a substrate (110), an anode layer (120), a semiconductor layer comprising a compound of Formula (1) (130), a photoactive layer (PAL) (151) and a cathode layer (190).

FIG. 2 is a schematic sectional view of an organic light-emitting diode (OLED) 100, according to an exemplary embodiment of the present invention. The OLED 100 includes a substrate (110), an anode layer (120), a semiconductor layer comprising a compound of Formula (1) (130), an emission layer (EML) (150) and a cathode layer (190).

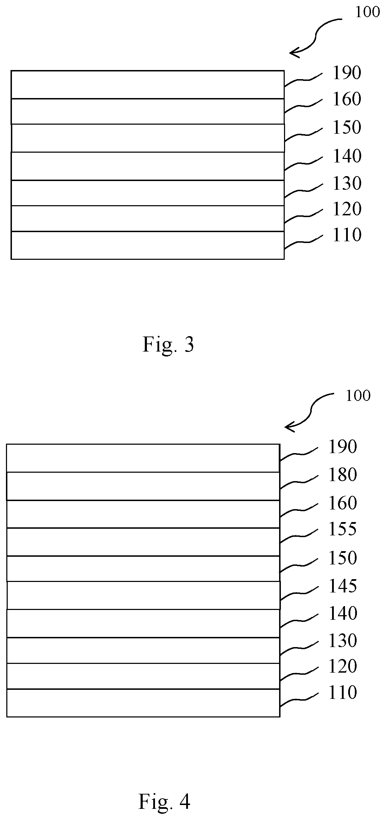

FIG. 3 is a schematic sectional view of an organic light-emitting diode (OLED) 100, according to an exemplary embodiment of the present invention. The OLED 100 includes a substrate (110), an anode layer (120), a semiconductor layer comprising a compound of Formula (1) (130), a hole transport layer (HTL) (140), an emission layer (EML) (150), an electron transport layer (ETL) (160) and a cathode layer (190).

FIG. 4 is a schematic sectional view of an organic light-emitting diode (OLED) 100, according to an exemplary embodiment of the present invention. The OLED 100 includes a substrate (110), an anode layer (120), a semiconductor layer comprising a compound of Formula (1) (130), a hole transport layer (HTL) (140), an electron blocking layer (EBL) (145), an emission layer (EML) (150), a hole blocking layer (HBL) (155), an electron transport layer (ETL) (160), an optional electron injection layer (EIL) (180), and a cathode layer (190).

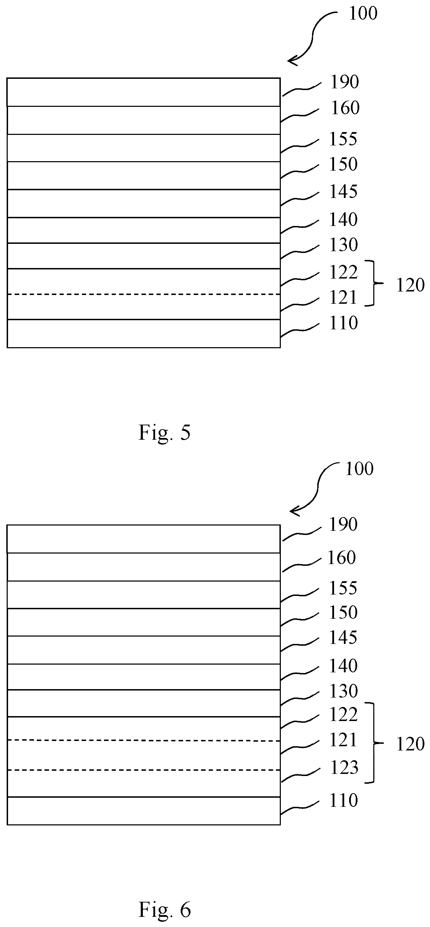

FIG. 5 is a schematic sectional view of an organic light-emitting diode (OLED) 100, according to an exemplary embodiment of the present invention. The OLED 100 includes a substrate (110), an anode layer (120) that comprises a first anode sub-layer (121) and a second anode sub-layer (122), a semiconductor layer comprising compound of Formula (1) (130), a hole transport layer (HTL) (140), an electron blocking layer (EBL) (145), an emission layer (EML) (150), a hole blocking layer (EBL) (155), an electron transport layer (ETL) (160) and a cathode layer (190).

FIG. 6 is a schematic sectional view of an organic light-emitting diode (OLED) 100, according to an exemplary embodiment of the present invention. The OLED 100 includes a substrate (110), an anode layer (120) that comprises a first anode sub-layer (121), a second anode sub-layer (122) and a third anode sub-layer (123), a semiconductor layer comprising compound of Formula (1) (130), a hole transport layer (HTL) (140), an electron blocking layer (EBL) (145), an emission layer (EML) (150), a hole blocking layer (EBL) (155), an electron transport layer (ETL) (160) and a cathode layer (190). The layers are disposed exactly in the order as mentioned before.

In the description above the method of manufacture an organic electronic device 101 of the present invention is for example started with a substrate (110) onto which an anode layer (120) is formed, on the anode layer (120), a semiconductor layer comprising compound of Formula (1) (130), a photoactive layer (151) and a cathode electrode 190 are formed, exactly in that order or exactly the other way around.

In the description above the method of manufacture an OLED 100 of the present invention is started with a substrate (110) onto which an anode layer (120) is formed, on the anode layer (120), a semiconductor layer comprising compound of Formula (1) (130), optional a hole transport layer (140), optional an electron blocking layer (145), an emission layer (150), optional a hole blocking layer (155), optional an electron transport layer (160), optional an electron injection layer (180), and a cathode electrode 190 are formed, exactly in that order or exactly the other way around.

The semiconductor layer comprising a compound of Formula (1) (130) can be a hole injection layer.

While not shown in Fig. 1 to Fig. 6, a capping layer and/or a sealing layer may further be formed on the cathode electrodes 190, in order to seal the OLEDs 100. In addition, various other modifications may be applied thereto.

Hereinafter, one or more exemplary embodiments of the present invention will be described in detail with, reference to the following examples. However, these examples are not intended to limit the purpose and scope of the one or more exemplary embodiments of the present invention.

### Detailed description

The invention is furthermore illustrated by the following examples which are illustrative only and non-binding.

Compounds of formula (1) may be prepared as described in WO2017029370A1 and WO2018150050A1.

### Sublimation temperature

Under nitrogen in a glovebox, 0.5 to 5 g compound are loaded into the evaporation source of a sublimation apparatus. The sublimation apparatus consist of an inner glass tube consisting of bulbs with a diameter of 3 cm which are placed inside a glass tube with a diameter of 3.5 cm. The sublimation apparatus is placed inside a tube oven (Creaphys DSU 05/2.1). The sublimation apparatus is evacuated via a membrane pump (Pfeiffer Vacuum MVP 055- 3C) and a turbo pump (Pfeiffer Vacuum THM071 YP). The pressure is measured between the sublimation apparatus and the turbo pump using a pressure gauge (Pfeiffer Vacuum PKR 251). When the pressure has been reduced to 10⁻⁵ mbar, the temperature is increased in increments of 10 to 30 K till the compound starts to be deposited in the harvesting zone of the sublimation apparatus. The temperature is further increased in increments of 10 to 30 K till a sublimation rate is achieved where the compound in the source is visibly depleted over 30 min to 1 hour and a substantial amount of compound has accumulated in the harvesting zone.

The sublimation temperature, also named T_{subl}, is the temperature inside the sublimation apparatus at which the compound is deposited in the harvesting zone at a visible rate and is measured in degree Celsius.

In the context of the present invention, the term "sublimation " may refer to a transfer from solid state to gas phase or from liquid state to gas phase.

### Decomposition temperature

The decomposition temperature, also named T_{dec}, is determined in degree Celsius.

The decomposition temperature is measured by loading a sample of 9 to 11 mg into a Mettler Toledo 100 µL aluminum pan without lid under nitrogen in a Mettler Toledo TGA-DSC 1machine. The following heating program was used: 25°C isothermal for 3 min; 25°C to 600°C with 10 K/min.

The decomposition temperature was determined based on the onset of the decomposition in TGA.

### Calculated HOMO and LUMO

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

### General procedure for fabrication of OLEDs

For Examples 1 to 21 and comparative examples 1 to 3 in Table 2, a glass substrate with an anode layer comprising a first anode sub-layer of 120 nm Ag, a second anode sub-layer of 8 nm ITO and a third anode sub-layer of 10 nm ITO was cut to a size of 50 mm x 50 mm x 0.7 mm, ultrasonically washed with water for 60 minutes and then with isopropanol for 20 minutes. The liquid film was removed in a nitrogen stream, followed by plasma treatment, see Table 2, to prepare the anode layer. The plasma treatment was performed in an atmosphere comprising 97.6 vol.-% nitrogen and 2.4 vol.-% oxygen.

Then, a substantially covalent matrix compound and a compound of formula (1) were codeposited in vacuum on the anode layer, to form a hole injection layer (HIL) having a thickness of 10 nm. The composition of the HIL can be seen in Table 2.

Then, the substantially covalent matrix compound was vacuum deposited on the HIL, to form a HTL having a thickness of 123 nm. The formula of the substantially covalent matrix compound in the HTL was identical to the substantially covalent matrix compound used in the HIL.

Then N-([1,1'-biphenyl]-4-yl)-9,9-diphenyl-N-(4-(triphenylsilyl)phenyl)-9H-fluoren-2-amine (CAS 1613079-70-1) was vacuum deposited on the HTL, to form an electron blocking layer (EBL) having a thickness of 5 nm.

Then 97 vol.-% H09 (Sun Fine Chemicals, Korea) as EML host and 3 vol.-% BD200 (Sun Fine Chemicals, Korea) as fluorescent blue emitter dopant were deposited on the EBL, to form a blue-emitting first emission layer (EML) with a thickness of 20 nm.

Then a hole blocking layer was formed with a thickness of 5 nm by depositing 2-(3'-(9,9-dimethyl-9H-fluoren-2-yl)-[1,1'-biphenyl]-3-yl)-4,6-diphenyl-1,3,5-triazine on the emission layer EML.

Then the electron transporting layer having a thickness of 31 nm was formed on the hole blocking layer by depositing 50 wt.-% 4'-(4-(4-(4,6-diphenyl-1,3,5-triazin-2-yl)phenyl)naphthalen-1-yl)-[1,1'-biphenyl]-4-carbonitrile and 50 wt.-% of LiQ.

Then Ag:Mg (90:10 vol.-%) was evaporated at a rate of 0.01 to 1 Å/s at 10⁻⁷ mbar to form a cathode layer with a thickness of 13 nm on the electron transporting layer.

Then, compound of formula F2 was deposited on the cathode layer to form a capping layer with a thickness of 75 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20°C. The current-voltage characteristic is determined using a Keithley 2635 source measure unit, by sourcing an operating voltage U in V and measuring the current in mA flowing through the device under test. The voltage applied to the device is varied in steps of 0.1V in the range between 0V and 10V. Likewise, the luminance-voltage characteristics and CIE coordinates are determined by measuring the luminance in cd/m² using an Instrument Systems CAS-140CT array spectrometer (calibrated by Deutsche Akkreditierungsstelle (DAkkS)) for each of the voltage values.

Lifetime LT of the device is measured at ambient conditions (20°C) and 30 mA/cm², using a Keithley 2400 sourcemeter, and recorded in hours. The brightness of the device is measured using a calibrated photo diode. The lifetime LT is defined as the time till the brightness of the device is reduced to 97 % of its initial value.

To determine the voltage stability over time U(50h)-(1h), a current density of at 30 mA/cm² was applied to the device. The operating voltage was measured after 1 hour and after 50 hours, followed by calculation of the voltage stability for the time period of 1 hour to 50 hours.

### Technical Effect of the invention

In order to investigate the usefulness of the inventive compounds, preferred materials were evaluated in view of their thermal properties.

**Table 1: Properties of compounds of formula (1) and comparative examples 1 to 4:**

| | Name | T_{dec} [°C] | T_{dec}-T_{subl} [°C] |
|---|---|---|---|
| Comparative compound 1 | Cu [TFSI]₂ | 180 | 5-10°C |
| Comparative compound 2 | Li [N(SO₂C₆F₁₃)₂] | - | Not Sublimable |
| Comparative compound 3 | Mg [N(SO₂ⁱC₃F₇)₂]₂ | >250 | ≥ 25 |
| Comparative compound 4 | Zn [N(SO₂ⁱC₃F₇)₂]₂ | >230 | ≥ 70 |
| A1 | 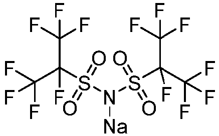 | >404°C | >101 |
| A2 | 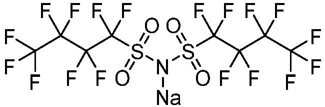 | >386 | >97 |
| A3 | 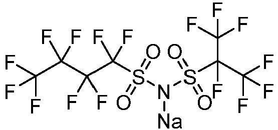 | >360 | >50 |
| A4 | 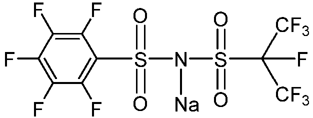 | >340 | >50 |
| A5 | 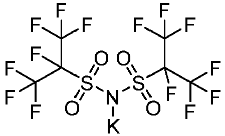 | >362 | >56 |
| A6 | 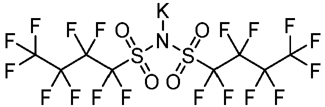 | >375 | >55 |
| A7 | 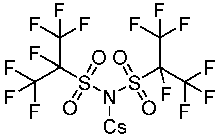 | >361 | >52 |
| A8 | 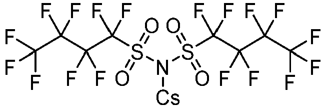 | >380 | >64 |
| A9 | 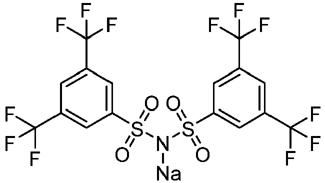 | >460 | >140 |
| A10 | 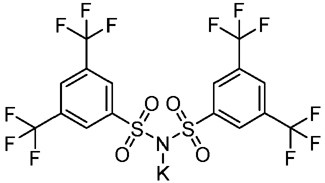 | >420 | >100 |
| A11 | 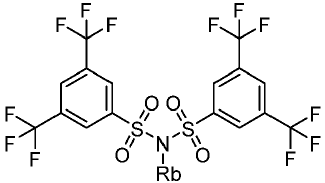 | >390 | >80 |
| A12 | 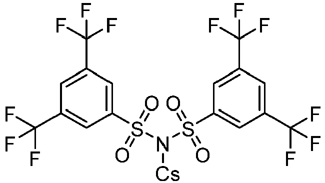 | >400 | >110 |

In Table 1 are shown the temperature at which thermal decomposition is observed (T_{dec}), difference between decomposition and sublimation temperature T_{dec}-T_{subl}.

The decomposition temperature of Cu (TFSI)₂ is 180 °C, see comparative example 1 in Table 1. The difference between decomposition and sublimation temperature is 10 °C. A sublimation rate which is suitable for mass production cannot be achieved as a substantial amount of compound decomposes before it sublimes.

In contrast, Li [N(SO₂C₆F₁₃)₂] is not sublimable, cf. comparative example 2 in Table 1.

Comparative example 3 comprises a magnesium complex. Comparative example 3 differs from comparative example 1 in the metal ion (Mg²⁺ instead of Cu²⁺) and the ligand (perfluorinated isopropyl groups instead of trifluoro methyl groups). The decomposition temperature is increased from 180 °C in comparative example 1 to > 250 °C. The difference between decomposition and sublimation temperature is ≥ 25 °C. The yield after sublimation is 80 %.

Comparative example 4 comprises a zinc complex. Comparative example 4 differs from comparative example 3 in the metal ion, namely Zn²⁺ instead of Mg²⁺. The difference between decomposition and sublimation temperature is further improved to ≥ 70 °C.

Compound A1 comprises a sodium complex. Compound A1 differs from comparative compound 2 in the metal ion (Na⁺ instead of Li⁺) and in the ligand (perfluorinated isopropyl groups instead of perfluorinated propyl groups). Compound A1 differs from comparative examples 3 and 4 in the metal ion (Na⁺ instead of Mg²⁺ or Zn²⁺). As can be seen in Table 1, the decomposition temperature is substantially increased. Additionally, the difference between decomposition and sublimation temperature is increased.

Compounds A2 to A4 differ from compound A1 in the ligands. As can be seen in Table 1, the thermal properties are improved over comparative compounds 1 to 4.

Compounds A5 to A8 differ from compounds A1 to A4 in the metal ion. As can be seen in Table 1, the thermal properties are improved over comparative compounds 1 to 4.

Compounds A9 to A12 differ from compounds A1 to A8 in the ligand and metal ion. As can be seen in Table 1, the thermal properties are improved over comparative compounds 1 to 4.

It is apparent that the inventive compounds show a higher decomposition temperature and/or a much larger gap between decay and sublimation temperature.

As materials for organic electronics are typically purified by sublimation, a high decomposition temperature, a large offset between decomposition and sublimation temperature and/or a high yield after sublimation are highly desirable. Thereby, a high sublimation rate may be achievable.

In Table 2, OLED performance data for examples 1 to 21 and comparative examples 1 to 3 are shown.

The performance of a semiconductor layer comprising a compound of formula (1) and a substantially covalent matrix compound of formula F2 or F1 was evaluated, see Table 2.

The HOMO of compound of formula F2 is -4.69 eV and the HOMO of compound of formula F1 is -4.81 eV, when calculated using TURBOMOLE V6.5 (TURBOMOLE GmbH, Litzenhardtstrasse 19, 76135 Karlsruhe, Germany) by applying the hybrid functional B3LYP with a 6-31G* basis set in the gas phase.

Due to the poor thermal properties of comparative compounds 1 and 2, OLED performance of compounds of formula (1) was assessed against comparative compounds 3 and 4.

**Table 2: Performance of organic electronic devices comprising compound of formula (1)**

| | Compound of formula (1) | Percentage of compound of formula (1) (vol.-%) | Matrix compound (cf. above) | Voltage U at 10 mA/cm² [V] | LT at 30 mA/cm² [h] | U(50h)-(1h) at 30 mA/cm² [V] |
|---|---|---|---|---|---|---|
| Comparative example 1 | Mg [N(SO₂ⁱC₃F₇)₂]₂ | 6 | F2 | 3.88 | 32 | 0.19 |
| Comparative example 2 | Zn [N(SO₂ⁱC₃F₇)₂]₂ | 6 | F2 | 3.85 | 10 | 0.29 |
| Example 1 | A1 | 6 | F2 | 3.85 | 74 | 0.03 |
| Example 2 | A2 | 6 | F2 | 3.84 | 67 | 0.01 |
| Example 3 | A3 | 6 | F2 | 3.82 | 79 | 0.02 |
| Example 4 | A4 | 6 | F2 | 3.83 | 82 | 0.01 |
| Example 5 | A5 | 6 | F2 | 3.83 | 56 | 0.05 |
| Example 6 | A6 | 6 | F2 | 3.85 | 53 | 0.06 |
| Example 7 | A7 | 6 | F2 | 3.84 | 63 | 0.04 |
| Example 8 | A8 | 6 | F2 | 3.84 | 61 | 0.06 |
| Example 17 | A9 | 4 | F2 | 3.90 | 62 | 0.013 |
| Example 18 | A10 | 4 | F2 | 3.78 | 92 | 0.026 |
| Example 19 | A11 | 4 | F2 | 3.91 | 70 | 0.024 |
| Example 20 | A11 | 6 | F2 | 3.89 | 74 | 0.018 |
| Example 21 | A12 | 4 | F2 | 3.85 | 86 | 0.105 |
| Comparative example 3 | Mg [N(SO₂ⁱC₃F₇)₂]₂ | 6 | F1 | 4.21 | 22 | 0.31 |
| Example 9 | A1 | 6 | F1 | 3.71 | 135 | 0.03 |
| Example 10 | A2 | 6 | F1 | 3.68 | 147 | 0.02 |
| Example 11 | A3 | 6 | F1 | 3.69 | 128 | 0.03 |
| Example 12 | A4 | 6 | F1 | 3.67 | 135 | 0.02 |
| Example 13 | A5 | 6 | F1 | 3.73 | 116 | 0.04 |
| Example 14 | A6 | 6 | F1 | 3.75 | 123 | 0.03 |
| Example 15 | A7 | 6 | F1 | 3.78 | 115 | 0.02 |
| Example 16 | A8 | 6 | F1 | 3.76 | 125 | 0.03 |

As can be seen in Table 2, OLED performance of a semiconductor layer comprising a compound of formula (1) may show reduced operating voltage U and/or improved lifetime and/or improved voltage stability over time compared to comparative compounds.

The particular combinations of elements and features in the above detailed embodiments are exemplary only; Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims. Furthermore, reference signs used in the description and claims do not limit the scope of the invention as claimed.

## Claims

1. An organic electronic device comprising an anode (120); a cathode (190); at least one photoactive laye (151) and at least one semiconductor layer (130), wherein the at least one semiconductor layer is arranged between the anode and the at least one photoactive layer; and wherein the at least one semiconductor layer comprises a compound of Formula (1): 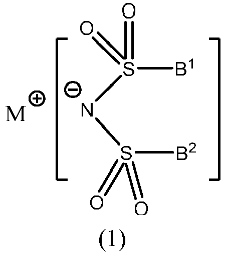 wherein
M is Na, K, Rb or Cs,
B¹ is selected from substituted or unsubstituted isopropyl, substituted or unsubstituted C₄ to C₁₂ alkyl, substituted or unsubstituted C₆ to C₁₂ aryl, substituted or unsubstituted C₂ to C₁₂ heteroaryl,
B² is selected from substituted or unsubstituted C₁ to C₁₂ alkyl, substituted or unsubstituted C₆ to C₁₂ aryl, substituted or unsubstituted C₃ to C₁₂ heteroaryl,
wherein the substituents on B¹ and B² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, halogen, F or CN;
wherein R¹ is selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy;
wherein at least one of the substituents on B¹ and/or B² is selected from C₃ to C₉ heteroaryl, C₁ to C₆ alkoxy, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkoxy, COR¹, COOR¹, halogen, F or CN.

2. The organic electronic device of Claim 1, whereby the substituents on B¹ and B² are independently selected from halogen, with F especially preferred, C₁ to C₃ perhalogenated, especially perfluorinated, alkyl or alkoxy, or -(O)ₗ-CₘH₂ₘ-CₙHalₙ₂ₙ₊₁ with l= 0 or 1, especially 0, m = 1 or 2, especially 1 and n = 1 to 3, especially 1 or 2 and Hal= halogen, especially F.

3. The organic electronic device of Claim 1 or 2, whereby at least one of B¹ and B² is substituted alkyl and the substituents of the alkyl moiety are fluorine with the number n_{F} (of fluorine substituents) and n_{H} (of hydrogens) fulfill the equation: n_{F} > n_{H} + 2.

4. The organic electronic device of Claim 1 or 3 whereby at least one of B¹ and B² is selected from perfluorinated alkyl or pentafluorophenyl.

5. The organic electronic device of Claim 1 or 4, whereby B¹ and B² are identical.

6. The organic electronic device of any of the Claims 1 to 4, whereby B¹ and B² are not identical.

7. The organic electronic device of any of the Claims 1 to 6, whereby the compound of formula (1) is free of alkoxy, COR¹ and/or COOR¹ groups.

8. The organic electronic device of any of the claims 1 to 7, whereby the compound of formula (1) contains 12 carbon atoms or less.

9. The organic electronic device of any of the claims 1 to 7, whereby the anion of compound (1) is selected from A-1 to A-53:

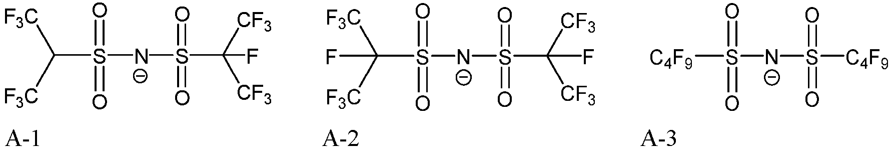

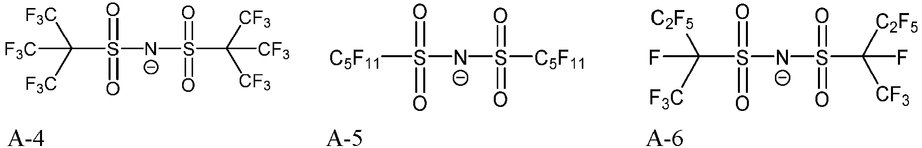

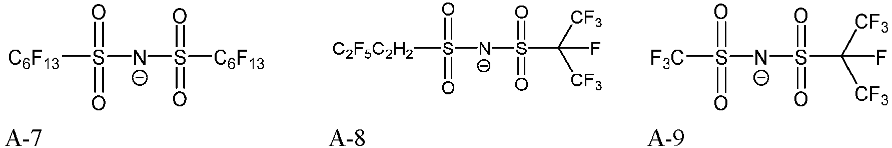

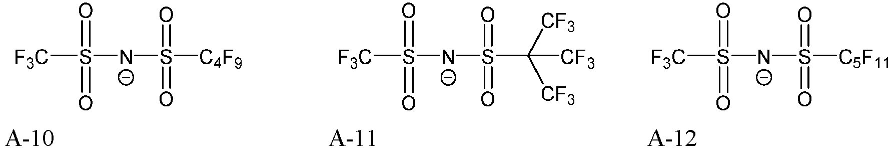

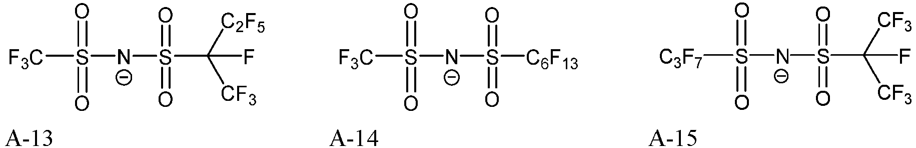

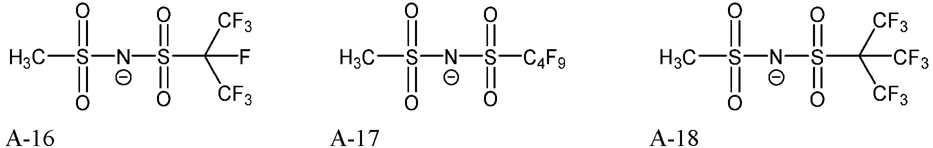

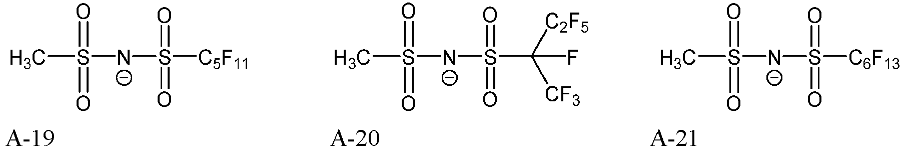

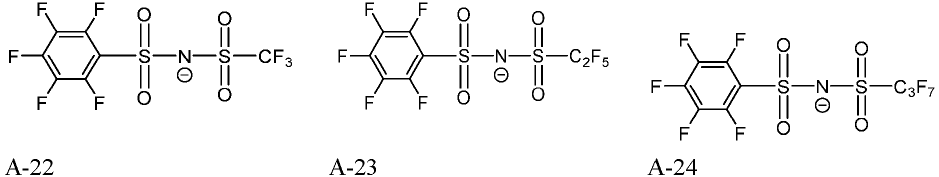

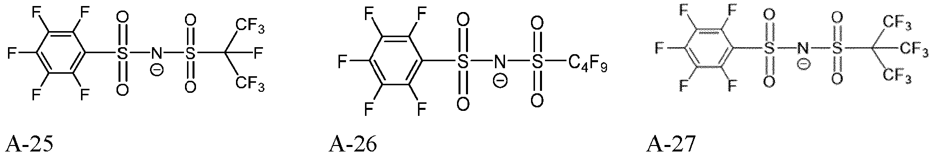

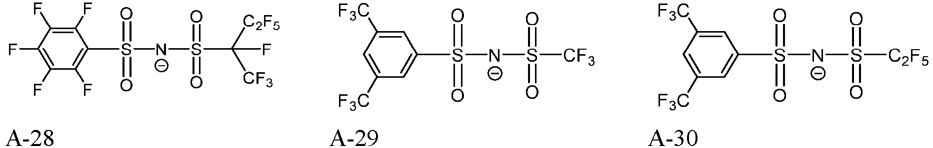

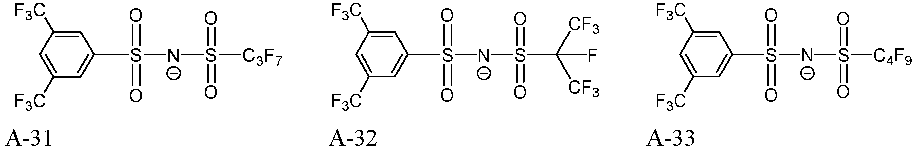

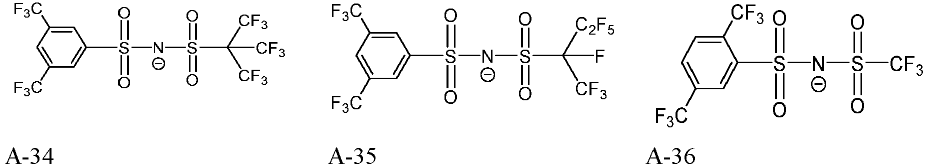

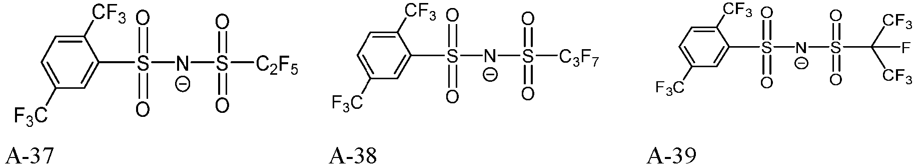

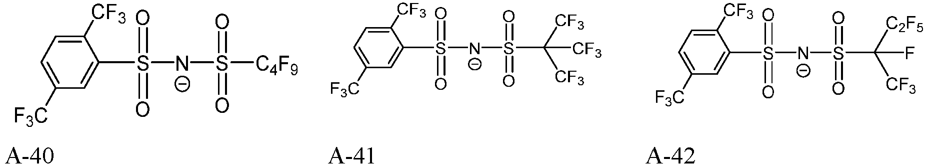

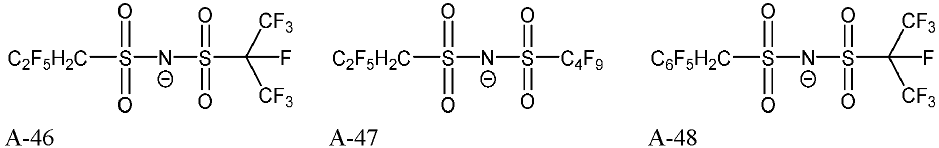

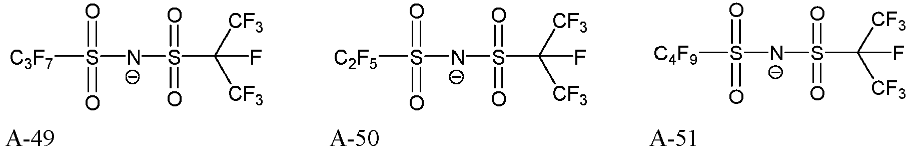

10. The organic electronic device of any of the claims 1 to 9, whereby the at least one semiconductor layer is non-emissive.

11. The organic electronic device of any of the claims 1 to 10, whereby at least one of the semiconductor layers is a hole-injection layer, which consists essentially of the compound of formula (1).

12. The organic electronic device of any of the claims 1 to 11, whereby at least one of the at least one semiconductor layers further comprises a substantially covalent matrix compound.

13. The electronic organic device of any of the claims 1 to 12, whereby the electronic organic device is an electroluminescent device, preferably an organic light emitting diode.

14. A display device comprising an organic electronic device according to any of the claims 1 to 13.

15. A compound of Formula (1): 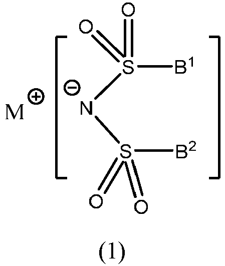 wherein Formula (1) is represented by one of the following formulae (1a) or (1b): 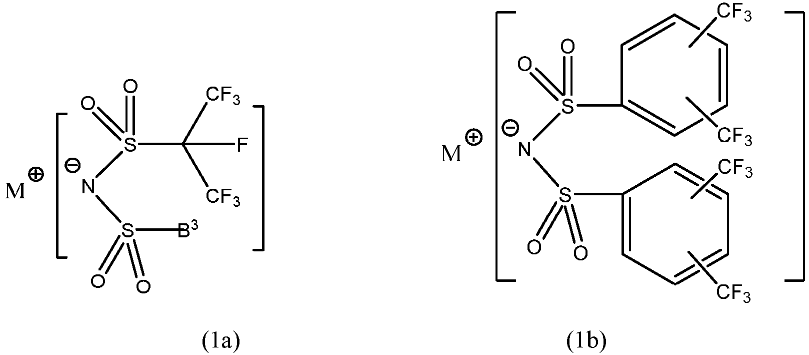 wherein
M is Na, K, Rb or Cs,
B³ is selected from substituted or unsubstituted C₁ to C₁₂ alkyl, substituted or unsubstituted C₆ to C₁₂ aryl, substituted or unsubstituted C₃ to C₁₂ heteroaryl,
wherein the substituents on B³ are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, halogen, F or CN;
wherein R¹ is selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy.

## Patentansprüche

1. Organische elektronische Vorrichtung, umfassend eine Anode (120); eine Kathode (190); mindestens eine photoaktive Schicht (151) und mindestens eine Halbleiterschicht (130), wobei die mindestens eine Halbleiterschicht zwischen der Anode und der mindestens einen photoaktiven Schicht angeordnet ist und wobei die mindestens eine Halbleiterschicht eine Verbindung der Formel (1) umfasst: 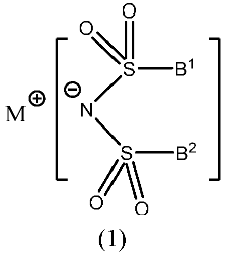 wobei
M für Na, K, Rb oder Cs steht,
B¹ aus substituiertem oder unsubstituiertem Isopropyl, substituiertem oder unsubstituiertem C₄- bis C₁₂-Alkyl, substituiertem oder unsubstituiertem C₆- bis C₁₂-Aryl, substituiertem oder unsubstituiertem C₂- bis C₁₂-Heteroaryl ausgewählt ist,
B² aus substituiertem oder unsubstituiertem C₁- bis C₁₂-Alkyl, substituiertem oder unsubstituiertem C₆- bis C₁₂-Aryl, substituiertem oder unsubstituiertem C₃- bis C₁₂-Heteroaryl ausgewählt ist,
wobei die Substituenten an B¹ und B² unabhängig aus D, C₆-Aryl, C₃- bis C₉-Heteroaryl, C₁- bis C₆-Alkyl, C₁- bis C₆-Alkoxy, verzweigtem C₃- bis C₆-Alkyl, cyclischem C₃-bis C₆-Alkyl, verzweigtem C₃- bis C₆-Alkoxy, cyclischem C₃- bis C₆-Alkoxy, teil- oder perfluoriertem C₁- bis C₁₆-Alkyl, teil- oder perfluoriertem C₁- bis C₁₆-Alkoxy, teil-oder perdeuteriertem C₁- bis C₆-Alkyl, teil- oder perdeuteriertem C₁- bis C₆-Alkoxy, COR¹, COOR¹, Halogen, F oder CN ausgewählt sind,
wobei R¹ aus C₆-Aryl, C₃- bis C₉-Heteroaryl, C₁- bis C₆-Alkyl, C₁- bis C₆-Alkoxy, verzweigtem C₃- bis C₆-Alkyl, cyclischem C₃- bis C₆-Alkyl, verzweigtem C₃- bis C₆-Alkoxy, cyclischem C₃- bis C₆-Alkoxy, teil- oder perfluoriertem C₁- bis C₁₆-Alkyl, teil- oder perfluoriertem C₁- bis C₁₆-Alkoxy, teil- oder perdeuteriertem C₁- bis C₆-Alkyl, teil- oder perdeuteriertem C₁- bis C₆-Alkoxy ausgewählt ist;
wobei mindestens einer der Substituenten an B¹ und/oder B² aus C₃- bis C₉-Heteroaryl, C₁- bis C₆-Alkoxy, verzweigtem C₃- bis C₆-Alkoxy, cyclischem C₃- bis C₆-Alkoxy, teil- oder perfluoriertem C₁- bis C₁₆-Alkyl, teil-oder perfluoriertem C₁- bis C₁₆-Alkoxy, teil- oder perdeuteriertem C₁- bis C₆-Alkoxy, COR¹, COOR¹, Halogen, F oder CN ausgewählt ist.

2. Organische elektronische Vorrichtung nach Anspruch 1, wobei die Substituenten an B¹ und B² unabhängig aus Halogen, wobei F besonders bevorzugt ist, perhalogeniertem, insbesondere perfluoriertem, C₁- bis C₃-Alkyl oder -Alkoxy oder -(O)ₗ-CₘH₂ₘ-CₙHalₙ₂ₙ₊₁ mit l = 0 oder 1, insbesondere 0, m = 1 oder 2, insbesondere 1, n = 1 bis 3, insbesondere 1 oder 2, und Hal = Halogen, insbesondere F, ausgewählt sind.

3. Organische elektronische Vorrichtung nach Anspruch 1 oder 2, wobei mindestens eines von B¹ und B² für substituiertes Alkyl steht und es sich bei den Substituenten der Alkylgruppierung um Fluor handelt, wobei die Zahl n_{F} (von Fluorsubstituenten) und die Zahl n_{H} (von Wasserstoffatomen) die folgende Gleichung erfüllen: n_{F} > n_{H} + 2.

4. Organische elektronische Vorrichtung nach Anspruch 1 oder 3, wobei mindestens eines von B¹ und B² aus perfluoriertem Alkyl oder Pentafluorphenyl ausgewählt ist.

5. Organische elektronische Vorrichtung nach Anspruch 1 oder 4, wobei B¹ und B² gleich sind.

6. Organische elektronische Vorrichtung nach einem der Ansprüche 1 bis 4, wobei B¹ und B² nicht gleich sind.

7. Organische elektronische Vorrichtung nach einem der Ansprüche 1 bis 6, wobei die Verbindung der Formel (1) frei von Alkoxy-, COR¹- und/oder COOR¹-Gruppen ist.

8. Organische elektronische Vorrichtung nach einem der Ansprüche 1 bis 7, wobei die Verbindung der Formel (1) 12 Kohlenstoffatome oder weniger enthält.

9. Organische elektronische Vorrichtung nach einem der Ansprüche 1 bis 7, wobei das Anion der Verbindung (1) aus A-1 bis A-53 ausgewählt ist:

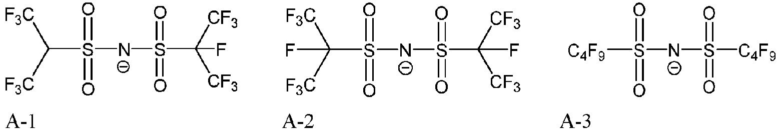

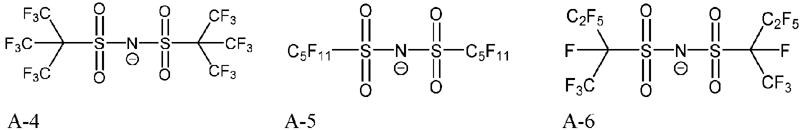

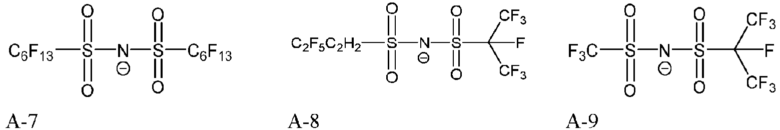

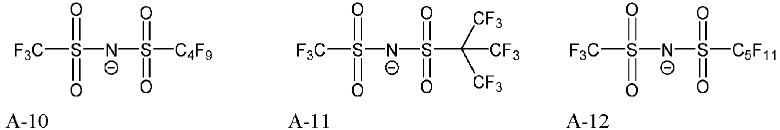

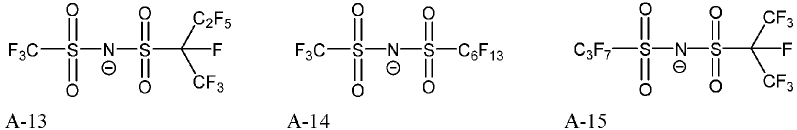

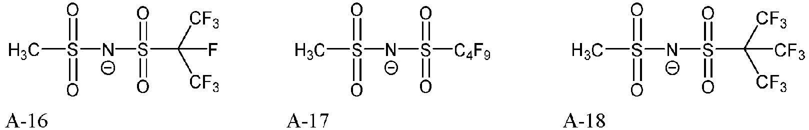

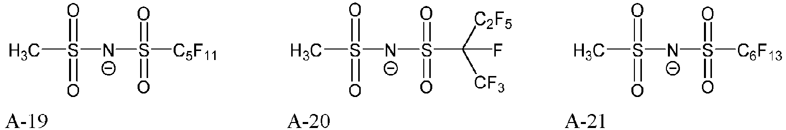

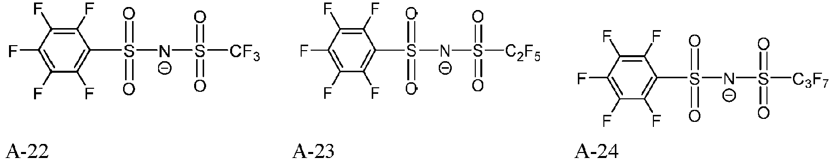

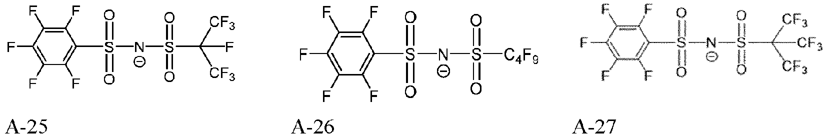

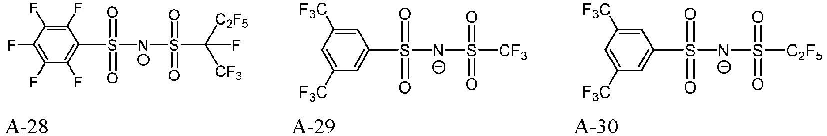

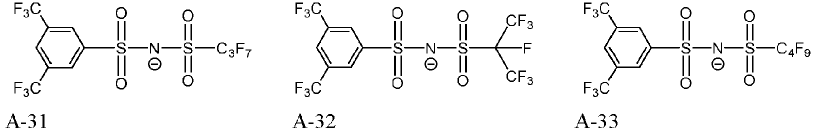

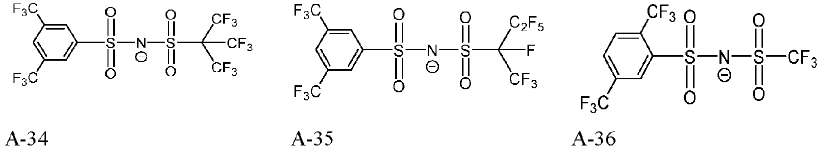

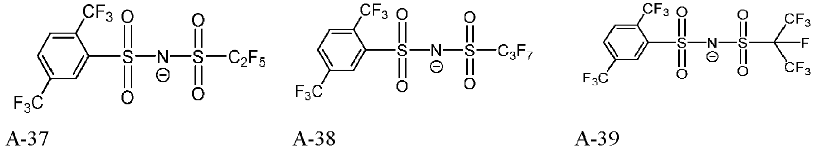

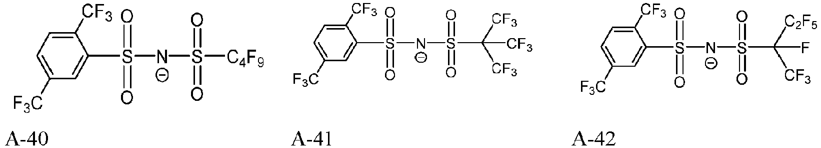

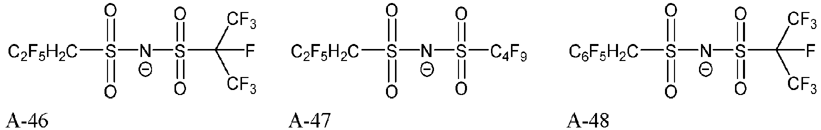

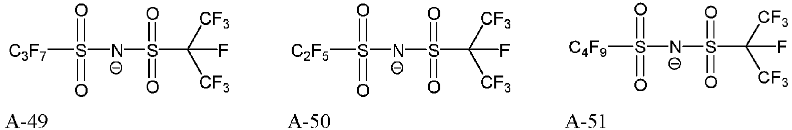

10. Organische elektronische Vorrichtung nach einem der Ansprüche 1 bis 9, wobei die mindestens eine Halbleiterschicht nichtemissiv ist.

11. Organische elektronische Vorrichtung nach einem der Ansprüche 1 bis 10, wobei mindestens eine der Halbleiterschichten eine Lochinjektionsschicht ist, die im Wesentlichen aus der Verbindung der Formel (1) besteht.

12. Organische elektronische Vorrichtung nach einem der Ansprüche 1 bis 11, wobei mindestens eine der mindestens einen Halbleiterschichten ferner eine weitgehend kovalente Matrixverbindung umfasst.

13. Elektronische organische Vorrichtung nach einem der Ansprüche 1 bis 12, wobei es sich bei der elektronischen organischen Vorrichtung um eine Elektrolumineszenzvorrichtung, vorzugsweise eine organische Leuchtdiode, handelt.

14. Anzeigevorrichtung, umfassend eine organische elektronische Vorrichtung gemäß einem der Ansprüche 1 bis 13.

15. Verbindung der Formel (1): 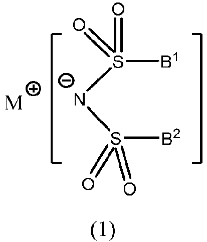 Wobei Formel (1) durch eine der folgenden Formeln (1a) oder (1b) wiedergegeben wird: 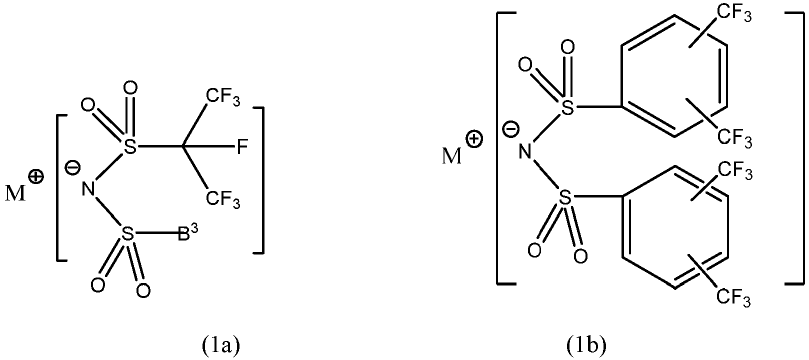 wobei
M für Na, K, Rb oder Cs steht,
B³ aus substituiertem oder unsubstituiertem C₁- bis C₁₂-Alkyl, substituiertem oder unsubstituiertem C₆- bis C₁₂-Aryl, substituiertem oder unsubstituiertem C₃- bis C₁₂-Heteroaryl ausgewählt ist,
wobei die Substituenten an B³ unabhängig aus D, C₆-Aryl, C₃- bis C₉-Heteroaryl, C₁- bis C₆-Alkyl, C₁- bis C₆-Alkoxy, verzweigtem C₃- bis C₆-Alkyl, cyclischem C₃- bis C₆-Alkyl, verzweigtem C₃- bis C₆-Alkoxy, cyclischem C₃- bis C₆-Alkoxy, teil- oder perfluoriertem C₁- bis C₁₆-Alkyl, teil-oder perfluoriertem C₁- bis C₁₆-Alkoxy, teil- oder perdeuteriertem C₁- bis C₆-Alkyl, teil- oder perdeuteriertem C₁- bis C₆-Alkoxy, COR¹, COOR¹, Halogen, F oder CN ausgewählt sind;
wobei R¹ aus C₆-Aryl, C₃- bis C₉-Heteroaryl, C₁- bis C₆-Alkyl, C₁- bis C₆-Alkoxy, verzweigtem C₃- bis C₆-Alkyl, cyclischem C₃- bis C₆-Alkyl, verzweigtem C₃- bis C₆-Alkoxy, cyclischem C₃- bis C₆-Alkoxy, teil- oder perfluoriertem C₁- bis C₁₆-Alkyl, teil- oder perfluoriertem C₁- bis C₁₆-Alkoxy, teil- oder perdeuteriertem C₁- bis C₆-Alkyl, teil- oder perdeuteriertem C₁- bis C₆-Alkoxy ausgewählt ist.

## Revendications

1. Dispositif électronique organique comprenant une anode (120) ; une cathode (190) ; au moins une couche photoactive (151) et au moins une couche de semi-conducteur (130), dans lequel l'au moins une couche de semi-conducteur est disposée entre l'anode et l'au moins une couche photoactive ; et dans lequel l'au moins une couche de semi-conducteur comprend un composé de Formule (1) : 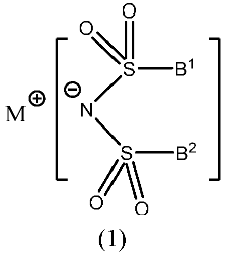 dans laquelle
M est Na, K, Rb ou Cs,
B¹ est choisi parmi isopropyle substitué ou non substitué, C₄ à C₁₂ alkyle substitué ou non substitué, C₆ à C₁₂ aryle substitué ou non substitué, C₂ à C₁₂ hétéroaryle substitué ou non substitué,
B² est choisi parmi C₁ à C₁₂ alkyle substitué ou non substitué, C₆ à C₁₂ aryle substitué ou non substitué, C₃ à C₁₂ hétéroaryle substitué ou non substitué,
dans laquelle les substituants sur B¹ et B² sont indépendamment choisis parmi D, C₆ aryle, C₃ à C₉ hétéroaryle, C₁ à C₆ alkyle, C₁ à C₆ alcoxy, C₃ à C₆ alkyle ramifié, C₃ à C₆ alkyle cyclique, C₃ à C₆ alcoxy ramifié, C₃ à C₆ alcoxy cyclique, C₁ à C₁₆ alkyle partiellement ou perfluoré, C₁ à C₁₆ alcoxy partiellement ou perfluoré, C₁ à C₆ alkyle partiellement ou perdeutéré, C₁ à C₆ alcoxy partiellement ou perdeutéré, COR¹, COOR¹, halogène, F ou CN ;
dans laquelle R¹ est choisi parmi C₆ aryle, C₃ à C₉ hétéroaryle, C₁ à C₆ alkyle, C₁ à C₆ alcoxy, C₃ à C₆ alkyle ramifié, C₃ à C₆ alkyle cyclique, C₃ à C₆ alcoxy ramifié, C₃ à C₆ alcoxy cyclique, C₁ à C₁₆ alkyle partiellement ou perfluoré, C₁ à C₁₆ alcoxy partiellement ou perfluoré, C₁ à C₆ alkyle partiellement ou perdeutéré, C₁ à C₆ alcoxy partiellement ou perdeutéré ;
dans laquelle au moins l'un des substituants sur B¹ et/ou B² est choisi parmi C₃ à C₉ hétéroaryle, C₁ à C₆ alcoxy, C₃ à C₆ alcoxy ramifié, C₃ à C₆ alcoxy cyclique, C₁ à C₁₆ alkyle partiellement ou perfluoré, C₁ à C₁₆ alcoxy partiellement ou perfluoré, C₁ à C₆ alcoxy partiellement ou perdeutéré, COR¹, COOR¹, halogène, F ou CN.

2. Dispositif électronique organique selon la revendication 1, dans lequel les substituants sur B¹ et B² sont indépendamment choisis parmi halogène, F étant notamment préféré, C₁ à C₃ alkyle ou alcoxy perhalogéné, notamment perfluoré, ou -(O)ₗ-CₘH₂ₘ-CₙHalₙ₂ₙ₊₁ où l = 0 ou 1, notamment 0, m = 1 ou 2, notamment 1 et n = 1 à 3, notamment 1 ou 2 et Hal = halogène, notamment F.

3. Dispositif électronique organique selon la revendication 1 ou 2, dans lequel au moins l'un parmi B¹ et B² est alkyle substitué et les substituants du groupement alkyle sont fluor, où le nombre n_{F} (de substituants fluor) et n_{H} (d'hydrogènes) satisfont l'équation : n_{F} > n_{H} + 2.

4. Dispositif électronique organique selon la revendication 1 ou 3, dans lequel au moins l'un parmi B¹ et B² est choisi parmi alkyle perfluoré ou pentafluorophényle.

5. Dispositif électronique organique selon la revendication 1 ou 4, dans lequel B¹ et B² sont identiques.

6. Dispositif électronique organique selon l'une quelconque des revendications 1 à 4, dans lequel B¹ et B² ne sont pas identiques.

7. Dispositif électronique organique selon l'une quelconque des revendications 1 à 6, dans lequel le composé de formule (1) est exempt de groupes alcoxy, COR¹ et/ou COOR¹.

8. Dispositif électronique organique selon l'une quelconque des revendications 1 à 7, dans lequel le composé de formule (1) contient 12 atomes de carbone ou moins.

9. Dispositif électronique organique selon l'une quelconque des revendications 1 à 7, dans lequel l'anion du composé (1) est choisi parmi A-1 à A-53 :

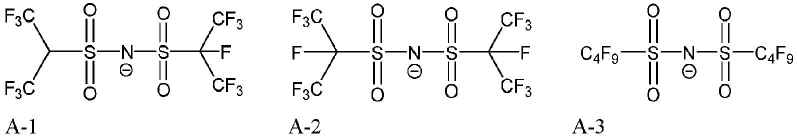

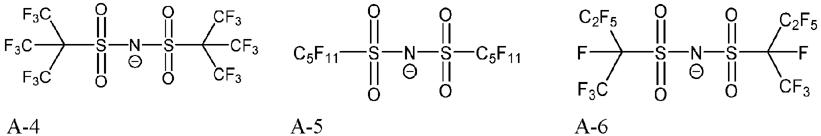

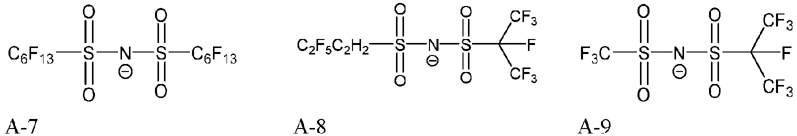

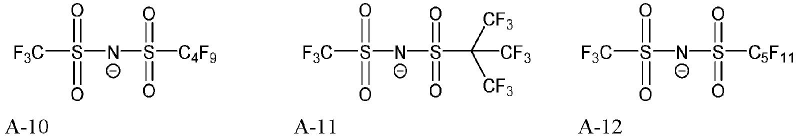

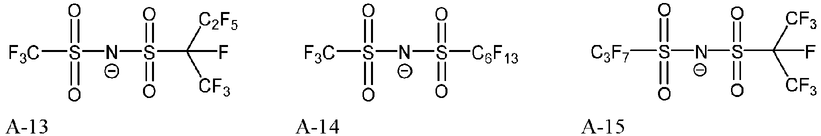

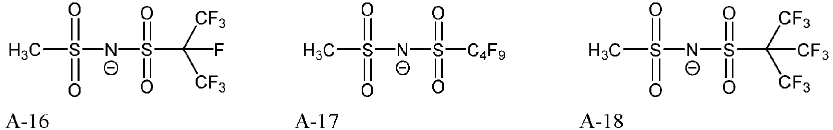

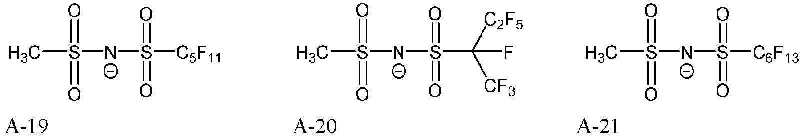

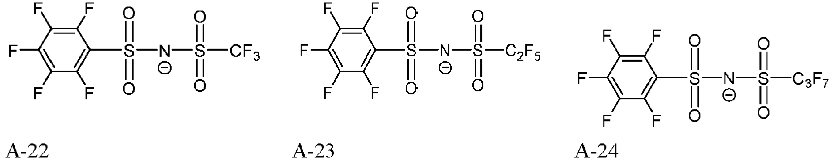

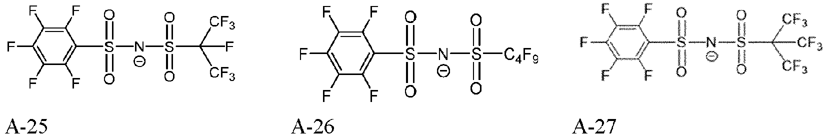

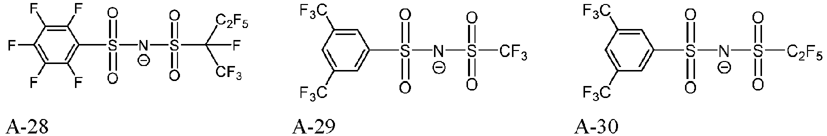

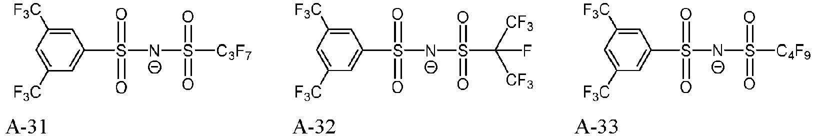

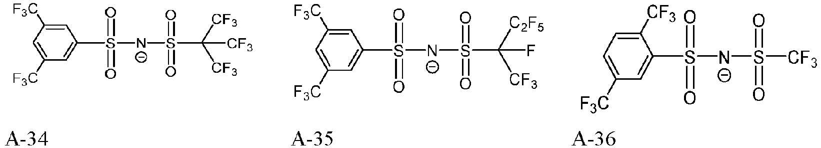

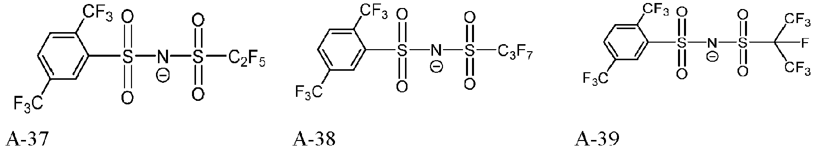

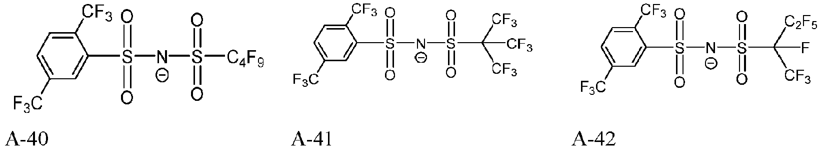

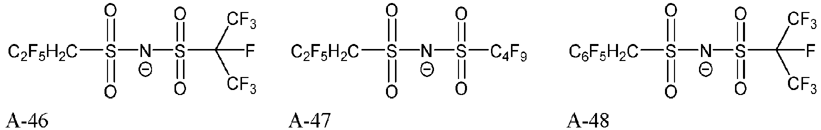

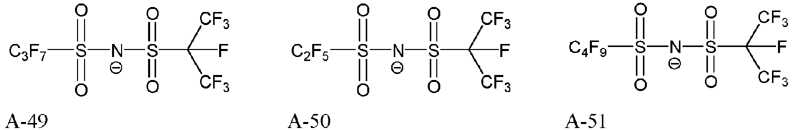

10. Dispositif électronique organique selon l'une quelconque des revendications 1 à 9, dans lequel l'au moins une couche de semi-conducteur est non émissive.

11. Dispositif électronique organique selon l'une quelconque des revendications 1 à 10, dans lequel au moins l'une des couches de semi-conducteur est une couche d'injection de trous, qui est essentiellement constituée du composé de formule (1).

12. Dispositif électronique organique selon l'une quelconque des revendications 1 à 11, dans lequel au moins l'une parmi les au moins une couche de semi-conducteur comprend en outre un composé matriciel sensiblement covalent.

13. Dispositif électronique organique selon l'une quelconque des revendications 1 à 12, dans lequel le dispositif électronique organique est un dispositif électroluminescent, de préférence une diode émettrice de lumière organique.

14. Dispositif d'affichage comprenant un dispositif électronique organique selon l'une quelconque des revendications 1 à 13.

15. Composé de Formule (1) : 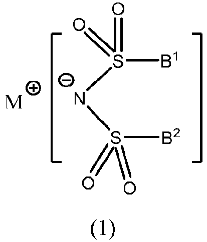 dans lequel la Formule (1) est représentée par l'une des formules (1a) ou (1b) suivantes : 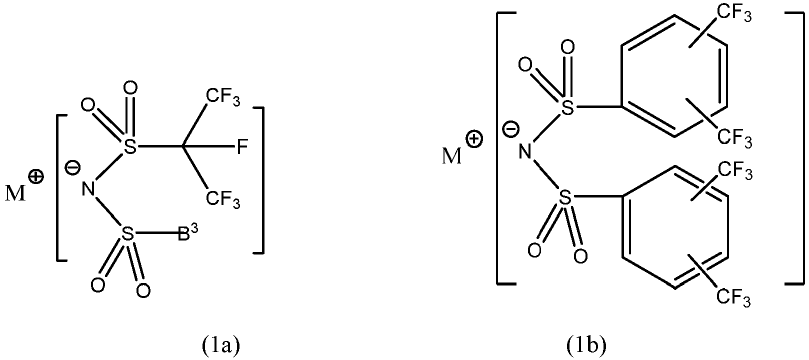 dans laquelle
M est Na, K, Rb ou Cs,
B³ est choisi parmi C₁ à C₁₂ alkyle substitué ou non substitué, C₆ à C₁₂ aryle substitué ou non substitué, C₃ à C₁₂ hétéroaryle substitué ou non substitué,
dans laquelle les substituants sur B³ sont indépendamment choisis parmi D, C₆ aryle, C₃ à C₉ hétéroaryle, C₁ à C₆ alkyle, C₁ à C₆ alcoxy, C₃ à C₆ alkyle ramifié, C₃ à C₆ alkyle cyclique, C₃ à C₆ alcoxy ramifié, C₃ à C₆ alcoxy cyclique, C₁ à C₁₆ alkyle partiellement ou perfluoré, C₁ à C₁₆ alcoxy partiellement ou perfluoré, C₁ à C₆ alkyle partiellement ou perdeutéré, C₁ à C₆ alcoxy partiellement ou perdeutéré, COR¹, COOR¹, halogène, F ou CN ;
dans laquelle R¹ est choisi parmi C₆ aryle, C₃ à C₉ hétéroaryle, C₁ à C₆ alkyle, C₁ à C₆ alcoxy, C₃ à C₆ alkyle ramifié, C₃ à C₆ alkyle cyclique, C₃ à C₆ alcoxy ramifié, C₃ à C₆ alcoxy cyclique, C₁ à C₁₆ alkyle partiellement ou perfluoré, C₁ à C₁₆ alcoxy partiellement ou perfluoré, C₁ à C₆ alkyle partiellement ou perdeutéré, C₁ à C₆ alcoxy partiellement ou perdeutéré.
